# FASCICULE DE BREVET EUROPEEN

(11) **EP 1 395 667 B1**
(45) Date de publication et mention de la délivrance du brevet: **08.11.2006**
(21) Numéro de dépôt: 02738252.2
(22) Date de dépôt: 16.05.2002
(51) Int. Cl.: C12N 15/82, A01H 5/00, A01H 5/10, C12Q 1/68

(54) **METHODE D'OBTENTION DE PLANTE MONOCOTYLEDONE CONTENANT UN GENE D'INTERET SANS SEQUENCE AUXILIAIRE ETRANGERE**
VERFAHREN ZUR HERSTELLUNG EINER MONOKOTYLEN PFLANZE, WELCHE EIN GEWÜNSCHTES GEN OHNE FREMDSEQUENZEN ENTHÄLT
METHOD FOR OBTAINING A MONOCOTYLEDON PLANT CONTAINING A GENE OF INTEREST FREE OF FOREIGN ANCILLARY SEQUENCE

(30) Priorité: 11.06.2001 FR 0107597
(43) Date de publication de la demande: 10.03.2004
(73) Titulaire: Biogemma, 75001 Paris (FR)
(72) Inventeur: PEREZ, Pascual, F-63450 Chanonat (FR); GERENTES, Denise, F-63450 Le Crest (FR); PRAUD, Sébastien, F-63130 Royat (FR)
(74) Mandataire: Flesselles, Bruno F.G.
(86) Numéro de dépôt international: PCT/FR2002/001656
(87) Numéro de publication internationale: WO 2002/101061

(56) Documents cités:
- WO-A-91/02071
- WO-A-92/01370
- WO-A-97/41228
- WO-A-98/38323

## Description

La présente invention concerne une méthode d'obtention de plante monocotylédone contenant un gène d'intérêt sans séquence auxiliaire étrangère.

La transgénèse végétale consiste à introduire dans une plante un ou des gènes provenant de différents organismes (bactéries, virus, insectes, plantes), dans le but de lui apporter de nouveaux caractères et d'améliorer certaines qualités agronomiques ou alimentaires. La grande diversité de gènes, associée au développement des techniques de transformation génétique classiques, a abouti à la création de nouvelles variétés végétales. Dans certains cas, grâce à l'introduction de caractères conférant une résistance à un herbicide, à des pathogènes ou à divers stress, les pratiques culturales peuvent être facilitées et les rendements augmentés. Dans d'autres cas, la valeur nutritive de la plante et la teneur en certains composés essentiels peuvent être améliorées. Cependant, l'intégration de gènes dans un génome durant un processus de transgénèse se fait avec une fréquence très basse. C'est pourquoi, dans la plupart des cas, on lie génétiquement aux gènes d'intérêts soit un gène marqueur de sélection, qui donne un avantage sélectif aux cellules transformées, soit un marqueur phénotypique, qui permet à l'homme de l'art d'identifier les cellules transformées parmi les autres.

Les gènes marqueurs de sélection, tels que les gènes de résistance aux antibiotiques ou aux herbicides, sont essentiels pour repérer les événements de transformation. Cependant ils demeurent dans la plante et par conséquent peuvent également être détectée sous forme d'ADN ou de protéines dans certains produits dérivés, alors que généralement ils n'apportent pas de valeur ajoutée à la plante transformée obtenue. La présence de ces gènes, et en particulier les gènes de résistance aux antibiotiques et aux herbicides, est aujourd'hui au centre de nombreux débats sur les organismes génétiquement modifiés (Flavell *et al.,* 1992 ; Chesson *et al.,* 2000).

Il est donc nécessaire d'élaborer des systèmes qui permettent l'élimination de ces gènes de sélection, une fois que les transformants ont été isolés par action de l'agent sélectif ou/et par analyses moléculaires. Plusieurs méthodes d'élimination plus ou moins complexes ont été étudiées, telles que :
- la co-transformation qui consiste à introduire deux ADN-T (ADN de transfert) dans l'organisme, le premier avec le gène d'intérêt et l'autre avec le gène de sélection. Cette méthode est plutôt utilisée pour les espèces à cycle de reproduction court. On sélectionne ensuite dans la descendance des transformants portant le transgène d'intérêt mais ayant perdu le gène de sélection par ségrégation.
- les systèmes exploitant la recombinaison à des sites spécifiques tels que le système *cre*/*lox* du bactériophage P1 ou le système FLP/FRT de la levure (FliPase recombinase ; Lyzrik et al., 1997) utilisés pour les espèces à cycle de reproduction long (maïs, arbustes). Mais ces systèmes restent complexes et lourds.
- les systèmes d'élimination qui exploitent les propriétés de gènes mobiles présents dans de nombreux génomes comme les rétroéléments et les transposons, et en particulier le système Ac/Ds du maïs, utilisés chez de nombreuses espèces.

Les éléments transposables,en général, dont notamment le système Ac/Ds, peuvent s'exciser et se réinsérer dans le génome pendant le développement cellulaire. Ces éléments peuvent aussi ne pas trouver de site receveur dans lequel s'insérer et sont alors dégradés par des nucléases. Principalement utilisés pour le clonage et l'étiquetage de certains gènes à phénotypes d'expression visibles (Dellaporta *et al.,* 1988), les transposons peuvent également servir à mobiliser, exciser et éliminer des gènes (PCT/US91/04679)*.*
Un système utilisant Ac/Ds du mais et permettant l'élimination de gènes marqueur de sélection chez la tomate, espèce qui ne possède pas d'élément Ac ou Ds à l'état naturel, a été décrit par Yoder *et al.,* (1993).

Mais ce système, qui a fait ses preuves dans des plantes hétérologues de type Dicotylédones (tomate, arabette, tabac), semblait jusqu'à lors difficilement exploitable chez des monocotylédones en particulier le maïs, espèce dans laquelle Ac/Ds réside naturellement. En effet la présence d'éléments endogènes rend difficile la maîtrise et l'exploitation de ce système. Ainsi, si WO 98/38323 suggère que ce système pourrait être utilisé dans le maïs, il m'exemplifie la mise en oeuvre que dans des plantes dicotylédones.

Dans le cadre de la présente demande, les inventeurs ont réussi à mettre au point un nouveau procédé original d'élimination de gènes marqueurs chez les monocotylédones, notamment chez le maïs, en utilisant un système de transposons endogène, comme par exemple le système Ac/Ds.

La présente invention a donc pour objet une méthode d'obtention d'une plante monocotylédone transgénique contenant un gène d'intérêt (i) sans séquence auxiliaire étrangère, comprenant les étapes suivantes:
a) transformation d'au moins une cellule de plante ne possédant aucune transposase active, avec un vecteur comprenant deux cassettes d'expression, l'une comprenant une séquence nucléotidique d'intérêt (i), l'autre comprenant une séquence nucléotidique codant pour un marqueur de sélection (ii) encadré par les séquences mobilisables d'un transposon, ladite cassette d'expression comprenant une séquence nucléotidique d'intérêt (i) étant en dehors de l'élément transposon;
b) sélection des plantes transformées avec le marqueur de sélection (ii);
c) croisement d'une plante transformée avec une autre plante appartenant à une lignée contenant dans son génome un gène, codant pour une transposase active endogène, et qui se trouve au milieu d'un marqueur phénotypique d'excision (iii), pour obtenir une F1 ou tout autre individu de génération ultérieure ;
d) sélection des cellules ou des individus porteurs du gène d'intérêt sans séquence auxiliaire étrangère, à partir de la génération F1 ;
e) régénération de plantes à partir des cellules ou des individus sélectionnés en (d).

Par «séquences auxiliaires étrangères», on entend des séquences (ii) codant pour un marqueur de sélection lequel peut être également un marqueur phénotypique permettant de sélectionner une plante transformée d'une plante qui ne contient pas l'ADN étranger transfecté. Comme séquence codant pour un marqueur de sélection, ou gène marqueur de sélection, on peut citer notamment un gène conférant une résistance à un antibiotique (Herrera-Estrella *et al.,* 1983), ou une résistance à un herbicide (EP 242 246), ou encore ;
- le gène sul conférant la résistance à l'herbicide sulfonamide Asulam (WO 98/49316),
- le gène nptll conférant la résistance à la kanamycine (Bevan *et al.,* 1983),
- le gène hph conférant la résistance à l'hygromycine (Herrera-Estrella *et al.,* 1983),
- le gène bar conférant la tolérance au bialaphos (White *et al.*, 1990),
- le gène EPSPS conférant la tolérance au glyphosate (US 5,188,642),
- le gène HPPD conférant la tolérance aux isoxazoles (WO 96/38567),
- le gène de la chloramphénicol acétyl transférase (CAT) qui détoxifie le chloramphénicol.

Dans le cadre de l'invention, on utilisera préférentiellement le gène bar ou le gène nptll.

Comme séquence codant pour un marqueur phénotypique, ou gène marqueur phénotypique utile comme marqueur de sélection (ii) dans le procédé selon l'invention, on peut citer les gènes rapporteurs permettant une sélection par un critère phénotypique, colorimétrique ou luminescent et facilement détectable, comme par exemple :
- le gène codant pour l'enzyme β-glucuronidase (GUS) (Jefferson *et al.,* 1987),
- le gène codant pour la protéine fluorescence verte GFP qui permet de visualiser, sous UV, les cellules transformées (Chalfie *et al.,* 1994), ainsi que toutes les autres protéines fluorescentes dérivées de la GFP,
- le gène nptll qui, utilisé à faible dose, inhibe la synthèse des chloroplastes,
ou plus généralement tout gène permettant l'expression d'un pigment comme par exemple les gènes intervenant dans la synthèse des flavonoïdes (anthocyanes, phlobaphènes et dérivés), qui peuvent être utilisés comme gènes rapporteurs dans la transformation des céréales. Ce système rapporteur permet l'expression de pigments allant du rouge au violet dans certains tissus transformés. On peut citer l'exemple des gènes régulateurs Lc ( Ludwig et al., 1990), B et C1 intervenant dans la synthèse des anthocyanes et le gène P intervenant dans la synthèse des phlobaphènes (Grotewold *et al.,* 1998).

Selon un mode préféré, on peut intégrer dans la cassette d'expression comprenant une séquence nucléotidique codant pour un marqueur de sélection (ii) un autre gène marqueur phénotypique de type gène rapporteur à sélection non destructive, pour faciliter les étapes de sélection par simple observation visuelle, non destructive. A titre d'exemple, on peut citer la GFP décrite par Nielsen et al. (1999) et tout autre variant de la GFP utilisable dans les plantes, comme par exemple ceux décrits par Seth J. Davis (1998).

Pour la séquence codant pour un marqueur de sélection (ii) ci-dessus, on utilisera avantageusement un promoteur spécifique d'un tissu ou d'un organe pour différencier l'expression de ce gène marqueur phénotypique (ii) par rapport à l'expression du marqueur phénotypique d'excision (iii) de la plante utilisée à l'étape (c) pour croiser la plante transformée.

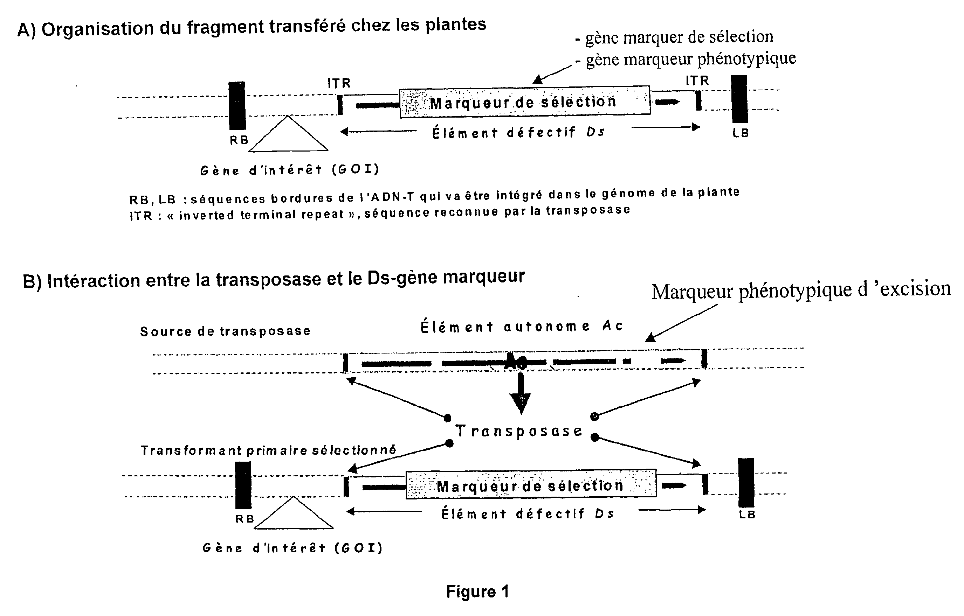

La présente invention concerne donc un système à deux composantes, qui sont représentées sur la figure n°1:
- une première plante ne possédant aucune transposase active bien que présentant un système transposon endogène, dans laquelle un construit, comprenant la cassette d'expression du gène d'intérêt (i) et celle du gène marqueur de sélection (ii), peut y être intégré.
- une deuxième plante contenant dans son génome un gène codant pour une transposase active endogène et qui est inséré dans un marqueur phénotypique d'excision (iii).

De façon préférentielle, la plante monocotylédone transformée selon l'invention ne contient aucune transposase active bien qu'ayant un système transposon endogène. De façon préférée, le plante monocotylédone transformée peut être une plante de maïs, et elle peut appartenir à :
- la lignée A188 qui ne contient aucune transposase active
- une lignée M1 sans aucune transposase active, issue du croisement des lignées A188 et B73, et favorable à la formation de cals de type II très régénérants
- ou plus généralement toute autre lignée ne contenant aucune transposase active.

Parmi les éléments transposables selon l'invention, on peut utiliser par exemple des familles de transposons mises en évidence chez les monocotylédones, telles que :
- l'élément *Dotted* (Dt), mis en évidence chez le maïs
- la famille d'éléments *Mutator* like (*Mu*), mise en évidence chez le maïs et le riz
- la famille *Activator*/*Dissociation* like (*Ac*/*Ds*), mise en évidence chez le maïs (Müller-Neumann *et al.*, 1984), l'orge (Chernyshev *et al.,* 1988), et le millet ( MacRae *et al.,* 1994)
- la famille CACTA like, mise en évidence chez le maïs (Pereira *et al.,* 1986), le sorgho (Chopra *et al.,* 1999), l'orge (Chernyshev *et al.,* 1988), et le riz (Mototashi R. *et al.,* 1996)
- la famille copia like, mise en évidence chez l'orge (Mannimem *et al.,* 1993)

Selon un mode préféré de l'invention, le système d'élimination, de gènes marqueurs est le système Ac/Ds de maïs.

Les éléments de la famille Activator/Dissociation (Ac/Ds) font partie des éléments transposables de type II, et se composent ainsi principalement de deux domaines :
- un gène qui code pour la protéine nécessaire à la mobilité des copies, la transposase.
- deux extrémités terminales inversées et répétées nommées ITR (Inverted Terminal Repeat). Les ITR et certaines séquences flanquantes semblent servir de repères pour diriger l'action de la transposase.
L'élément Ds est un élément Ac qui a subi d'importantes mutations ou délétions dans la séquence codant pour la transposase. Il ne peut s'exciser de son site d'insertion qu'en présence d'une source de transposase active *Ac.* Il est donc *Ac*-dépendant.
Par extension on peut appeler un élément Ds tout élément composé de deux ITR entourant n'importe quelle séquence interne dont la taille est inférieure à celle de l'élément autonome.

Selon la présente invention, le construit d'ADN utilisé à l'étape (a) comprend une cassette d'expression du gène d'intérêt (i) et une cassette d'expression du gène marqueur de sélection (ii).

Le gène marqueur à éliminer (ii) est intégré entre deux bordures provenant des extrémités d'un élément *Ac* et contenant les séquences nécessaires à la transposition. Le tout est inséré dans l'ADN-T qui porte ainsi un élément transposon défectif de type Ds contenant le gène de sélection. Cet élément est nommé Ds::M (M pour Marqueur de sélection ou Marqueur phénotypique).

Le gène d'intérêt est quant à lui sur l'ADN-T et en dehors de l'élément transposable Ds::M.

Selon la présente invention, les séquences nucléotidiques d'intérêt (i) peuvent être toutes les séquences d'acides nucléiques permettant de donner ou améliorer un trait de caractère intéressant chez la plante transgénique résultante. Par exemple, la séquence d'acide nucléique peut coder pour des protéines ou des transcrits ARN antisens qui peuvent permettre d'améliorer des valeurs nutritionnelles, le rendement, la résistance aux pathogènes, aux maladies, à la sécheresse.... De tels gènes sont notamment décrits dans les demandes de brevet WO 91/02071 et WO 95/06128.

Les séquences nucléiques d'intérêt peuvent également être introduites en tant qu'outil génétique pour générer des mutants et/ou assister l'identification, le marquage moléculaire ou l'isolement de segments de gènes de plantes. D'autres exemples sont décrits dans Weising *et al.,* 1995.

Selon la présente invention, l'acide nucléique d'intérêt (i) est inséré dans une construction d'acides nucléiques, appelée cassette d'expression du gène d'intérêt, et est lié de manière opérationnelle à des éléments permettant son expression et éventuellement sa régulation.

Parmi ces éléments, on peut citer les promoteurs, les activateurs et les terminateurs de transcription.

Parmi les promoteurs de transcription pouvant être utilisés, on peut citer notamment des promoteurs constitutifs, des promoteurs spécifiques de tissus ou d'organes, ainsi que des promoteurs spécifiques du développement.

De manière générale, on utilisera préférentiellement un promoteur constitutif tel que le promoteur pAct 1 du gène Act 1 de riz contenu dans le plasmide pAct1-F4 (Mc Elroy *et al.*, 1991) ou le promoteur p35S (Kay *et al.*, 1987), ou un promoteur tissu spécifique comme le promoteur HMWG de blé ou d'orge, ou encore le promoteur pCRU du gène de la cruciférine de radis, qui permettent tous trois une expression de la protéine d'intérêt dans les graines (Roberts *et al.,* 1989 ; Anderson O.D. *et al.,* 1989 ; Depigny-This *et al.,* 1992).

D'autres éléments tels que les introns, enhancers, séquences de polyadénylation et dérivées, peuvent également être présents dans la séquence nucléique d'intérêt, pour améliorer l'expression ou le fonctionnement du gène transformant.
De manière avantageuse, la cassette d'expression peut aussi contenir des séquences 5' non traduites dites 'leader'. De telles séquences peuvent améliorer la traduction.

Parmi les terminateurs utilisables dans les constructions de l'invention, on peut citer notamment : le terminateur Nos correspondant à la région 3' non-codante du gène de la nopaline synthase du plasmide Ti d'*Agrobacterium tumefaciens* (Depicker *et al.,* 1982).

La cassette d'expression du gène d'intérêt (i) est insérée dans un vecteur nucléotidique, tel qu'un plasmide, qui comprend en outre la cassette d'expression du gène marqueur (ii) inséré entre deux ITR.

Selon la présente invention, la cassette d'expression du gène marqueur (ii) comprend l'acide nucléique codant pour le marqueur de sélection ou le marqueur phénotypique, flanqué des éléments nécessaires à son expression, notamment les promoteurs, les activateurs et les terminateurs de transcription, tels que décrits ci-dessus.

Selon un mode de réalisation du procédé d'invention, les cellules végétales sont transformées par un vecteur tel que défini précédemment, transféré dans un hôte cellulaire susceptible d'infecter lesdites cellules végétales en permettant l'intégration dans le génome de ces dernières, des séquences nucléotidiques d'intérêt initialement contenues dans le génome du vecteur susmentionné. Avantageusement, l'hôte cellulaire utilisé est une souche bactérienne, telle que *Agrobacterium tumefaciens,* notamment selon la méthode décrite dans l'article d'An *et al.,* (1986), ou encore *Agrobacterium rhizogenes,* notamment selon la méthode décrite dans l'article de Guerche *et al.,* (1987).

Par exemple, la transformation des cellules végétales peut être réalisée par le transfert de la région T du plasmide circulaire extra chromosomique indicateur de tumeurs Ti d'*Agrobacterium tumefaciens,* en utilisant un système binaire (Watson *et al.,* 1994). Pour ce faire, deux vecteurs sont construits. Dans l'un de ces vecteurs, la région T a été éliminée par délétion, à l'exception des bordures gauche et droite, un gène marqueur étant inséré entre eux pour permettre la sélection dans les cellules de plantes. L'autre partenaire du système binaire est un plasmide Ti auxiliaire, plasmide modifié qui n'a plus de région T mais qui contient toujours les gènes de virulence *vir*, nécessaire à la transformation de la cellule végétale.

Selon un mode préféré, on peut utiliser la méthode décrite par lshida *et al.* (1996), pour la transformation des monocotylédones.

On peut citer aussi d'autres modes de réalisation du procédé de l'invention, notamment les méthodes de transfert direct de gènes aux cellules végétales telles que la microinjection directes dans des embryoïdes de plante (Neuhaus *et al.,* 1987), l'infiltration in planta des inflorescences (Bechtold *et al.,* 1993) ou l'électroporation de protoplastes (Chupeau *et al.,* 1989) ou encore la précipitation directe au moyen de PEG de protoplastes (Schocher *et al.,* 1986) ou le bombardement de cals ou d'embryons par canon de particules recouvertes de l'ADN plasmidique d'intérêt (Fromm M. *et al.,* 1990).

Selon un autre protocole, la transformation est réalisée selon la méthode décrite par Finer *et al.* (1992), utilisant le canon à particules de tungstène ou avantageusement d'or, sur des cals ou des embryons.

La sélection à l'étape (b) des cellules de plantes transformées peut être effectuée sur milieu sélectif ou par simple observation visuelle dans le cas de l'utilisation d'un gène marqueur phénotypique.

Le croisement de l'étape (c) s'effectue entre la plante transformée et une autre plante, ayant une transposase active endogène et qui est au milieu d'un marqueur phénotypique d'excision (iii), pour obtenir une F1 ou tout autre individu de génération ultérieure selon les techniques connues de l'homme du métier. L'autre plante est préférentiellement une lignée élite à caractères agronomiques d'intérêt.

Selon la présente invention, on entend par transposase active endogène une transposase active obtenue de façon non transgénique.

Selon la présente invention, la plante transformée et identifiée est croisée à l'étape (c) avec une autre plante appartenant à une lignée contenant dans son génome un gène, codant pour une transposase active endogène, et qui se trouve insérée dans un marqueur phénotypique d'excision (iii).

Les transposons ont tendance à se méthyler au cours des générations, en particulier chez le maïs, plus aucun transcrit n'est alors détectable. La phase active du transposon correspond donc à un état de faible méthylation qui permet la transcription normale du gène. En associant au transposon un marqueur phénotypique d'excision on peut visualiser les cellules dans lesquelles il y a eu transposition et quantifier le niveau d'activité du transposon dans l'organisme et ainsi visualiser la production de transposase. Le transposon est généralement inséré entre le promoteur et la partie codante du marqueur phénotypique d'excision, rendant impossible son expression. Lorsque le transposon exprime la transposase, il peut s'exciser du gène dans lequel il était inséré, ce qui restaure l'activité du gène. On peut ainsi visualiser par analyse phénotypique les cellules dans lesquelles il y a eu production de la transposase susceptible d'exciser l'élément Ds::M.

Par « marqueur phénotypique d'excision » (iii), on entend tout gène codant pour un pigment ou tout autre gène permettant de suivre l'évolution de l'excision de l'élément Ac. A titre d'exemple un marqueur phénotypique d'excision peut être tout gène dont une mutation donne un phénotype visible sur la graine ou la plante ou une partie de la plante par exemple des gènes intervenant dans la voie biochimique des anthocyanes ou des phlobaphènes. On peut citer les gènes constitutifs A1, A2, C2, Bz1ou Bz2 et les gènes régulateurs C1, Vp1, Pl, P1 et R1. Selon la présente invention l'allèle préférentiel peut être l'allèle R-nj qui est un marqueur phénotypique endogène de la famille R1 présent dans certaines lignées de maïs. L'allèle R-nj::transposon est porteur d'un élément Ac actif, la région chromosomique à laquelle appartient R-nj étant particulièrement hypométhylée (Brink *et al.,* 1973). Lorsque l'Ac se transpose hors du gène R-nj, ce dernier redevient généralement fonctionnel, ce qui se traduit par la production de secteurs anthocyanés violet de type "navajo" visibles sur la couronne de la graine, dont l'embryon. Ce phénotype variégué est un bon moyen pour évaluer et localiser des événements pour lesquels il y a eu production de transposase. En outre, il permet de vérifier l'activité du transposon au cours des générations et de suivre la transposase au fil des croisements.

A titre d'exemple de lignées utilisables à l'étape (c) contenant dans son génome un gène codant pour une transposase active endogène, et qui se trouve insérée dans un marqueur phénotypique d'excision, on peut citer les lignées :
- W22/R-nj::Ac, lignée homozygote pour l'allèle R-nj : :Ac issue de graines provenant du Stock Center (http://www.agron.missouri.edu/).
- A188/R-nj::Ac, lignée homozygote pour l'allèle R-nj : :Ac introgressé dans le fond génétique de A188.
- Hill/R-nj::Ac lignée homozygote pour l'allèle R-nj : :Ac introgressé dans le fond génétique de Hill.
- W23/P-vv, lignée homozygote pour l'allèle P-vv issue de graines provenant du Stock Center. Un élément Ac est inséré dans l'allèle P-rr.
- ou plus généralement toute lignée possédant un transposon actif inséré dans un gène à phénotype visible et possédant le génotype nécessaire à son expression.

L'allèle porteur de l'élément Ac, bien qu'instable peut-être transféré à d'autres lignées par plusieurs croisements. Il suffit de vérifier avant l'introgression que la lignée réceptrice possède bien la totalité des allèles nécessaires à l'expression du phénotype. Dans le cas de l'allèle R-nj::Ac, les allèles A1, A2, Bz1, Bz2 et C1 doivent être présents ou peuvent être introgressés.

La sélection, à l'étape (d), des individus qui portent le gène d'intérêt (i) sans séquence auxiliaire étrangère peut être réalisée sur des plantes F1, ou des plantes F2, ou des cals issus d'embryons F1. La sélection des individus peut être appréciée par des techniques connues de l'homme du métier comme les techniques de PCR et de Southern, ou d'observations phénotypiques. Il est donc effectué par exemple une identification des événements transformés ayant intégré dans leur génome l'ADN-T porteur du gène marqueur de sélection (ii), et une sélection des événements monocopie en particulier. Il est possible d'effectuer une sélection des plantes F1 résistantes pour rechercher des excisions somatiques, et une analyse des plantes F2 sensibles pour détecter d'éventuelles excisions germinales.

La présente invention concerne également une méthode d'obtention d'une plante Monocotylédone transgénique contenant un gène d'intérêt sans séquence auxiliaire étrangères dans laquelle la sélection à l'étape (d) des individus se fait par voie reproductive et comprend les étapes suivantes :
- sélection des grains F1 variégués ;
- sélection des événements d'excision somatique de l'élément DS : :M par technique PCR ;
- sélection des événements d'excision germinale de l'élément Ds : :M par technique PCR;
- obtention de semis de plantes F2 à partir de ces événements.

Ainsi selon la présente invention la sélection des individus porteurs du gène d'intérêt (i) sans séquence auxiliaire étrangère peut être issue d'une analyse de la variégation de l'allèle marqueur d'excision du transposon, sur les épis F1.

Entre également dans le cadre de l'invention une méthode d'obtention d'une plante monocotylédone transgénique contenant un gène d'intérêt (i) sans séquence auxiliaire étrangère dans laquelle la sélection des individus, à l'étape (d), se fait par voie végétative et comprend les étapes suivantes :
- production de cals à partir d'embryons immatures F1
- sélection visuelle des cals contenant l'ADN-T et l'élément Ds : :M
- multiplication de cals et recherche de secteurs d'excision de l'élément Ds : :M
- régénération de plantes F1 à partir de ces secteurs d'excision.

Selon la présente invention, des plantes F1 peuvent être obtenues directement par régénération à partir de cals sélectionnés à l'étape (d), par la voie de culture *in vitro* d'embryons immatures d'épis F1.

L'invention permet d'obtenir des plantes monocotylédones transgéniques ou parties de plantes, telles que cellules ou cals, contenant un gène d'intérêt sans séquences auxiliaires.

Les plantes monocotylédones hybrides, caractérisées en ce qu'elles sont obtenues par le croisement des plantes obtenues par les procédés décrits ci-dessus, peuvent aussi être obtenu, via un procédé selon l'invention.

Ceci concerne en particulier les plantes de maïs.

L'invention utilise une méthode de sélection sur cals des événements présentant une excision de l'élément Ds : :M pour l'obtention de plantes transgéniques contenant un gène d'intérêt (i) sans séquence auxiliaire étrangère, comprenant les étapes :
- production de cals à partir d'embryons immatures F1
- sélection visuelle des cals contenant l'ADN-T et l'élément Ds : :M
- multiplication de cals et recherche de secteurs d'excision de l'élément Ds : :M
- régénération de plantes F1 à partir de ces secteurs d'excision.

La formation de cal est due à une prolifération cellulaire, c'est à dire un amas de cellules indifférenciées et rejuvénilisées qui pourront donner naissance à des embryons bipolaires. Ces embryons bipolaires peuvent, sous certaines conditions connues de l'homme de l'art, se comporter comme des embryons zygotiques - issus de la fécondation entre une cellule sexuelle femelle et une cellule sexuelle mâle - et donner une plante.

Lors des divisions cellulaires qui ont lieu durant de la croissance du cal, un certain nombre d'événements d'excision du transposon Ds::M vont avoir lieu. Le cal ainsi constitué est donc chimérique et contient des secteurs de cellules possédant le Ds::M et des secteurs de cellules ne possédant plus le Ds::M.

Le gène marqueur phénotypique permet d'identifier les cellules transformées au cours des processus de transgénèse qui possèdent un phénotype pouvant s'exprimer dans le cal.

Alternativement, cette méthode peut également servir à reconnaître les cellules dans lesquelles le gène marqueur phénotypique (iii) ne s'exprime plus, soit à cause d'une extinction du gène, soit à cause de l'excision du gène. On choisira préférentiellement un gène marqueur phénotypique de type GFP, anthocyanes ou tout autre gène marqueur à expression colorimétrique. On peut également utiliser le gène nptll qui inhibe la synthèse des chloroplastes.

L'invention utilise un vecteur comprenant deux cassettes d'expression comme décrites précédemment. Une cellule hôte,

par exemple une cellule de plante, ou un clone d'une telle cellule, transformée par un vecteur comme décrit précédemment peut être obtenue.

La présente invention peut également s'appliquer à d'autres plantes, notamment monocotylédones, qui possèdent:
- un système transposon comme décrit précédemment, et
- un système marqueur phénotypique ; par exemple de type anthocyane, retrouvé chez les Poaceae (maïs, sorgho, blé, avoine, riz),
qui sont les deux composantes essentielles du procédé selon l'invention. Parmi les monocotylédones qui peuvent également être obtenues selon le même protocole, on peut citer par exemple ; *Sorghum bicolor* (sorgho), *Hordeum vulgare* (orge), *Triticum eastivum* (blé), *Oriza sativa* (riz), *Pennisetum glaucum* (millet).

### LEGENDE DES FIGURES

**Figure n°1** : schéma des deux composantes du système : le Ds : :M à éliminer et la source de transposase.
**Figures n°2 :** cartes de restriction des plasmides pBios340-nptII (figure 2A) et pBios342-bar (figure 2B), dérivés du plasmide pSB12.

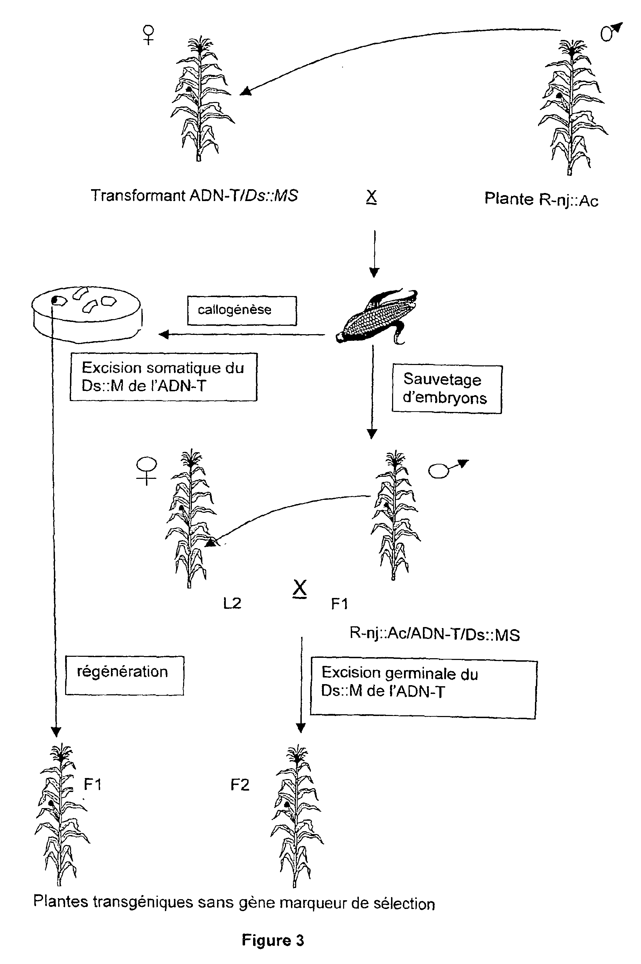

**Figure n°3 :** schéma des deux voies possibles pour obtenir des plantes transgéniques sans gène marqueur de sélection.

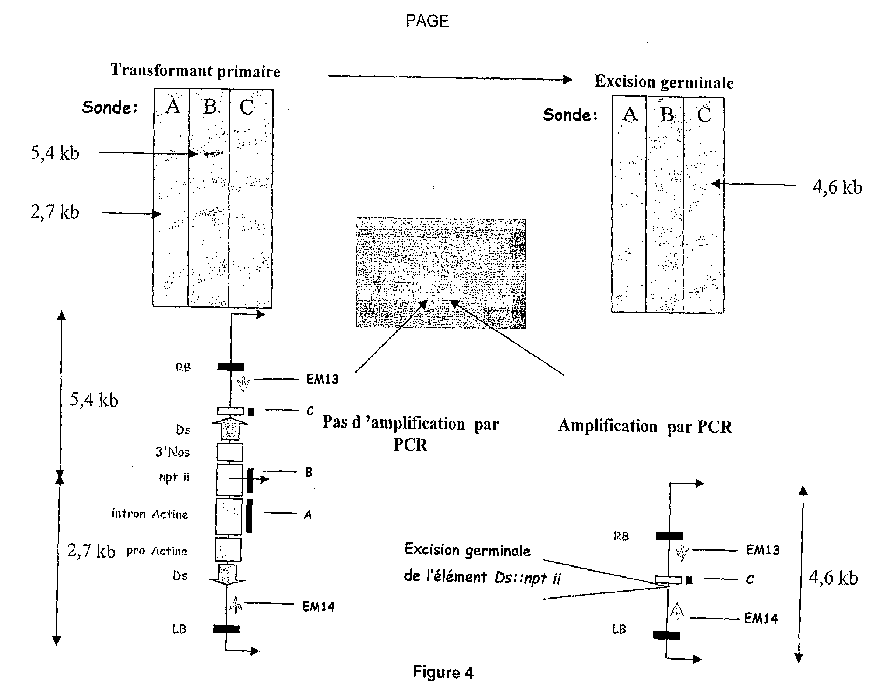

**Figure n°4 :** observation de l'excision germinale
**Figure n°5 :** carte de restriction du plasmide pBios491 comprenant le gène GFP et le gène NptII dans un élément Ds.
**Figure n°6 :** carte de restriction du plasmide pBios 424 comprenant le promoteur A9-barnase-terminateur 3' CaMV et le gène de résistance à la kanamycine NptII dans un élément dissociant Ds.

### EXEMPLES

### Exemple 1. Obtention d'une lignée A188, Ac inactive, transformée avec l'élément Ds::M de façon stable

### 1.1. Préparation des construits Ds::M

L'élément Ac dont la séquence a servi à faire les construits Ds : :M est l'Ac inséré dans le locus p (Lechelt *et al.,* 1989) identique à l'Ac9 du gène waxy (Fedoroff *et al.* 1983). Les pieds du Ds::M ont ensuite été construits par délétions internes de l'Ac. Leurs tailles sont: - 337 pb pour extrémité 3' Ac
- 404 pb pour extrémité 5' Ac

L'exemple proposé dans cette invention traite de deux vecteurs qui présentent un polylinker dans un construit d'ADN, permettant ainsi d'amener un gène d'intérêt par clonage.

Les transformations des plantes ont été effectuées avec deux vecteurs pBios340 et pBios342, décrits ci-dessous, issus du même vecteur de départ pSB12 (Japan Tobacco, EP 672 752), contenant les deux bordures LB et RB.
A l'intérieur de l'élément Ds, un gène marqueur de sélection (MS) a été introduit sous contrôle du promoteur du gène actine 1 de riz, suivi du premier intron actine de riz (Mc Elroy *et al.,* 1991).

### a) Ds : :npt II (pBios340)

Le plasmide pBios340 contient un ADN-T constitué des bordures de pSB12, de l'extrémité 5' de l'élément Ac, le promoteur et le premier intron du gène Actine 1 de riz, le cadre de lecture ouvert npt II (Bevan *et al.,* 1983), le terminateur Nos (du gène qui code pour la nopaline synthase isolé chez *Agrobacterium tumefaciens*) et l'extrémité 3' de l'élément Ac.

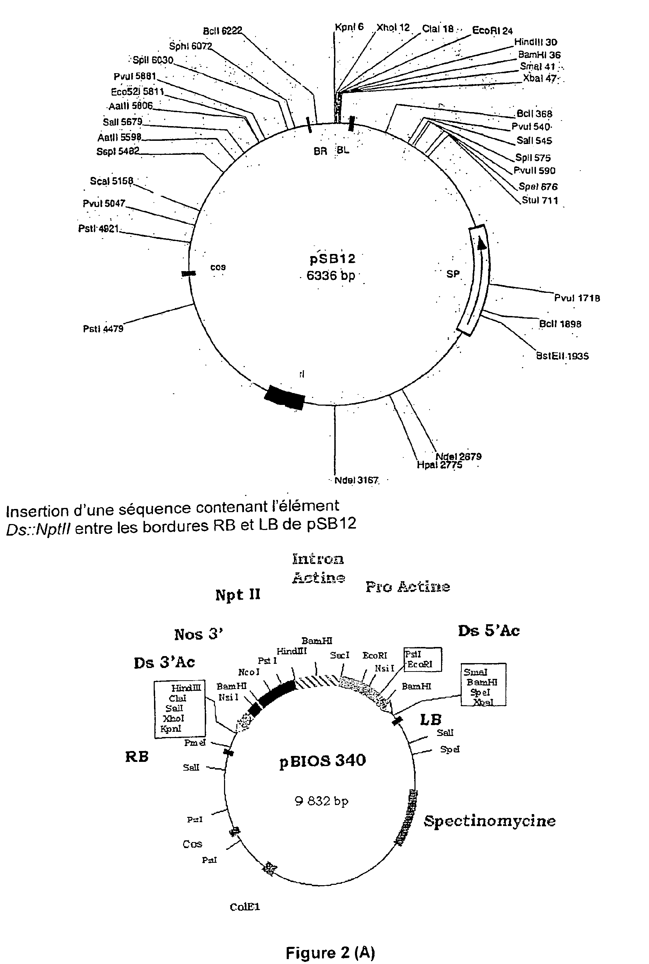

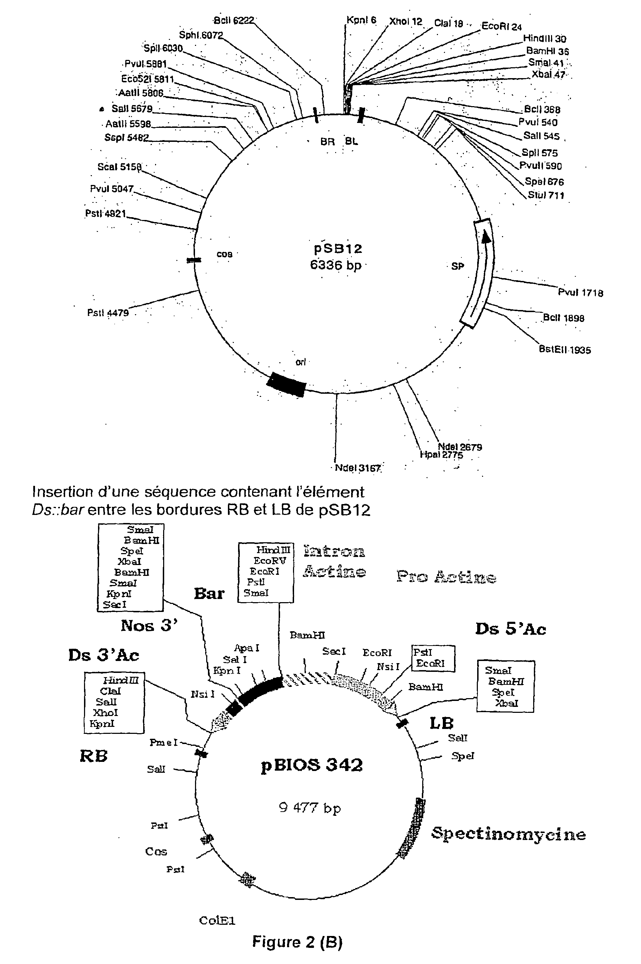

Le Ds : :npt II a une taille de 3428 pb (figure n°2).

### b) Ds : :bar (pBios342)

Le plasmide pBios342 a la même organisation que pBios340 et possède le cadre de lecture ouverte bar (White *et al.,* 1990), à la place du cadre de lecture ouverte npt II (figure n°2)

Le Ds : :bar a une taille de 3173 pb.

Pour simplifier, la cassette d'expression promoteur - intron actine - cadre de lecture ouverte npt II ou bar-terminateur Nos sera considéré comme un gène fonctionnel npt II ou bar.

### 1.2 Transformation du maïs et régénération

### a) choix d'une lignée ne possédant aucune transposase Ac active

Les lignées, qui peuvent être utilisées, doivent préférentiellement ne comporter aucune transposase active pour que l'ADN-T soit stable dans la plante transformée, et avoir une bonne aptitude à la transformation par *Agrobacterium tumefaciens.*

On utilise ainsi la lignée A188, qui est une lignée de l'espèce *Zea mays subsp mays.*

Afin d'évaluer l'activité des transposases endogènes, l'état de méthylation des éléments Ac est analysé.

L'ADN de chaque plante a été digéré par les endonucléases BglII et PvuII séparément, puis par un mélange de ces deux enzymes.

PvuII est une enzyme sensible à la méthylation. Une fois que le résidu Cytosine de son site spécifique de coupure est méthylé (5'-C-A-G-^{m}C-T -G-3'), l'enzyme ne peut plus couper la séquence. Cette enzyme permet de révéler un état de méthylation de la séquence. Elle possède seulement deux sites de coupures dans la séquence de l'élément Ac qui appartiennent tous deux à deux régions riches en dinucléotides CpG. Ces îlots sont sensibles à la méthylation chez les plantes. Le fragment PvuII-PvuII de Ac fait 2524 pb.

BglII est une endonucléase qui ne possède aucun site à l'intérieur de l'élément Ac mais assez fréquent dans le génome du maïs.

Les produits de digestion d'ADN génomique ont été séparés par électrophorèse en gel d'agarose et transférés sur membranes nylon.
Une sonde SAc de 1605 pb a été obtenue par digestion de l'élément Ac par l'endonucléase HindIII. Ce fragment HindIII-HindIII correspond à une partie interne de l'élément Ac et ne peut donc pas s'hybrider sur les éléments Ds trop délétés par rapport à Ac et ne possédant plus cette partie d'Ac. L'hybridation est donc limitée aux éléments transposables et/ou aux séquences présentant une bonne homologie avec l'élément Ac. Cette sonde provient d'un plasmide qui contient l'élément autonome Ac9 initialement inséré dans le locus p du maïs. Les fragments de 1605 pb ont été extraits du gel d'agarose après digestion et migration. Ils ont ensuite été purifiés avant d'être hybridés sur les membranes nylon.

Seules des bandes de tailles supérieures à 8 kb sont présentes en haut de chaque piste pour les échantillons d'ADN de A188 et d'une lignée élite digérés par PvuII et BglII/PvuII ; aucune bande de taille proche de 2,5 kb n'est visible. Ce profil de digestion montre que l'ADN génomique a été partiellement digéré par PvuII. L'absence de cette bande de 2524 pb dans les plantes A188 peut être due à un état de méthylation des transposases Ac qui seraient donc inactives ou bien à l'absence de copie complète d'un élément Ac dans ces fonds génétiques.

En outre la lignée A188 contient tous les gènes constitutifs nécessaires (A1, A2, Bz1, Bz2, C2) et les allèles récessifs des gènes régulateurs de la voie de synthèse des anthocyanes (r-r,C1,pl). Elle n'exprime donc aucun pigment de type anthocyane, sauf dans les anthères de la gaine des feuilles qui peut parfois être rouge. C'est donc une lignée réceptrice intéressante pour l'expression du marqueur anthocyane après croisement.

### b) transformation par Agrobacterium et régénération

La technique de transformation utilisée est celle décrite par Ishida *et al.,* 1996. *Agrobacterium fumefaciens* a été utilisé, notamment à partir d'embryons immatures de 10 jours après la fécondation. Tous les milieux utilisés sont donnés dans la référence citée. La transformation débute avec une phase de co-culture où les embryons immatures des plantes de maïs sont mis en contact pendant au moins 5 jours avec une souche d'*Agrobacterium tumefaciens,* comme LBA 4404 (Ooms *et al.,* 1981) ou autre EHA 101 (Hood *et al.,* 1986), EHA105 ( Hood *et al.*, 1993), AGL1 (Lazo *et al.,* 1991) contenant les vecteurs superbinaires. Le plasmide superbinaire est le résultat d'une recombinaison homologue entre un vecteur intermédiaire porteur de l'ADN-T contenant le gène d'intérêt et/ou le marqueur de sélection dérivé des plasmides décrits dans les exemples précédents, et le vecteur pSB1 de Japan Tobacco (EP 672 752) qui contient : les gènes virB et virG du plasmide pTiBo542 présent dans la souche supervirulente A281 d'*Agrobacterium tumefaciens* (ATCC 37349) et une région homologue retrouvée dans le vecteur intermédiaire permettant cette recombinaison homologue. Les embryons sont ensuite placés sur milieu LSAs pendant 3 jours à l'obscurité et à 25°C. Une première sélection est effectuée sur les cals transformés : les cals embryogènes sont transférés sur milieu LSD5 contenant de la phosphinotricine à 5 mg/l et de la céfotaxime à 250 mg/l (élimination ou limitation de la contamination par *Agrobacterium tumefaciens*). Cette étape est menée 2 semaines à l'obscurité et à 25°C. La deuxième étape de sélection est réalisée par transfert des embryons qui se sont développés sur milieu LSD5, sur milieu LSD10 (phosphinotricine à 10 mg/l) en présence de céfotaxime, pendant 3 semaines dans les mêmes conditions que précédemment. La troisième étape de sélection consiste à exciser les cals de type 1 (fragments de 1 à 2 mm) et à les transférer 3 semaines à l'obscurité et à 25°C sur milieu LSD 10 en présence de céfotaxime.

La régénération des plantules est effectuée en excisant les cals de type 1 qui ont proliféré et en les transférant sur milieu LSZ en présence de phosphinotricine à 5 mg/l et de céfotaxime pendant 2 semaines à 22°C et sous lumière continue.

Les plantules ayant régénéré sont transférées sur milieu RM + G2 contenant 100mg/l de céfotaxime pendant 2 semaines à 22°C et sous illumination continue pour l'étape de développement. Les plantes obtenues sont alors transférées au phytotron en vue de leur acclimatation en serre.

### 1.3 Sélection de transformants résistants et analyse moléculaire

### a) sélection sur milieu sélectif

### Sélection des événements de transformation résistants au glufosinate (Ds :: bar)

Plusieurs milieux sélectifs, le milieu d'initiation de cals, de maturation, et de régénération permettent d'éliminer efficacement les événements non transformés sensibles à l'agent sélectif. Seuls les cals transformés sont régénérés et peuvent donner des plantules Afin d'éliminer les plantules issues d'un échappement éventuel et qui se sont développées au voisinage de plantes résistantes (le gène *bar* produit une protéine qui permet à la plante de détoxifier le glufosinate), un badigeonnage est réalisé sur une partie d'une feuille de la plante (Leaf painting assay) au stade six feuilles avec une solution contenant du glufosinate (Basta 0,5 % (v/v)). La feuille sensible présente, une semaine plus tard, un dessèchement caractéristique.

### Sélection des événements de transformation résistants à la kanamycine (Ds :: nptII)

La sélection des événements de transformation a été réalisée sur des milieux contenant différentes concentrations de kanamycine ou de généticine.
Un test de repérage des plantes transformées en serre a également été mis en place. Quelques gouttes d'une solution de kanamycine (500 mg/l) et de Tween 20 (0,1 % (p/v)) sont déposées dans le cornet que forment les feuilles des jeunes plantes (stade trois feuilles, environ deux à trois semaines). Les feuilles des plantes sensibles présentent en grandissant des secteurs blanchis à l'endroit où elles ont été en contact avec la solution. Ce test est non destructif et permet de récupérer les plantes sensibles.

### b) analyse moléculaire

Les plantes régénérées sont analysées par PCR et par Southern blot afin d'éliminer les échappements et d'identifier les événements de transformation monocopies qui seront utilisées dans cette invention de façon préférentielle. Ces plantes ayant intégré l'ADN-T dans leur génome sont les transformants primaires T0. La technique d'analyse utilisée est dérivée de celle décrite par Southern (1976).

### Identification par PCR

Des PCR ont été réalisées sur de l'ADN extrait de ces plantes, afin de vérifier que les plantes résistantes aux agents sélectifs ont bien intégré dans leur génome l'ADN-T et qu'il n'y a pas eu de transposition du *Ds::M*.
Le couple d'oligonucléotides EM13-EM14 (SEQ ID n°1 - SEQ ID n°2) amplifie un fragment dont la taille est 280 pb quand le Ds::M s'est excisé de l'ADN-T et aucun fragment si le Ds::M est toujours inséré dans son site initial sur l'ADN-T.
Le couple d'oligonucléotides Actint1-Actint2 (SEQ ID n°3 - SEQ ID n°4) amplifie un fragment de l'intron actine présent dans le Ds::MS dont la taille est 480 pb.
Les événements de transformation sélectionnés sur milieu sélectif, positifs à la PCR avec le couple Actint1-Actint2 et négatifs à la PCR avec le couple EM13-EM14 ont donc intégré dans leur génome au moins une partie de l'ADN-T contenant l'élément Ds::M qui est resté dans son site d'insertion initial.

### Détermination du nombre de copies de l'ADN-T

Des analyses moléculaires ont été effectuées sur l'ADN extrait de feuilles des plantes sélectionnées afin de déterminer les événements de transformation monocopies. L'ADN des plantes positives Ds : :bar a été digéré par l'endonucléase *Eco*RV qui ne coupe qu'une seule fois dans l'ADN-T entre le cadre de lecture ouverte bar et l'intron Actine.

L'ADN des plantes positives a été digéré par l'endonucléase *N*col qui ne coupe qu'une seule fois dans l'ADN de transfert à l'intérieur du cadre de lecture ouverte npt II.

Après électrophorèse, des transferts ont été effectués et les membranes résultantes ont été hybridées successivement avec les sondes suivantes.
Pour les plantes Ds : :bar, des sondes Bar (gène Bar digéré par Smal, nommée S006), intron Actine (nommée S063) et 5'Nos (nommée S064).
Pour les plantes Ds : :nptII, des sondes npt II (nommée S020), intron Actine (nommée S063) et 5'Nos (nommée S064).
Les sondes intron Actine, 5'Nos et nptll sont définies par PCR à partir d'oligonucléotides choisis selon les méthodes connues de l'homme du métier (cf. tableau annexe)

Les sondes Bar, npt II et intron Actine permettent d'estimer le nombre de copies d'ADN de transfert inséré dans le génome. La sonde 5'Nos permet de mettre en évidence une excision potentielle de l'élément Ds de l'ADN-T. Ces trois sondes permettent donc de déterminer les plantes possédant une seule copie de l'ADN de transfert complet.

Pour Ds : :bar, lorsque l'insertion de l'ADN-T est unique, un fragment est révélé pour chacune des trois sondes. De plus si le Ds n'est pas excisé, la taille des fragments d'ADN révélés par les deux sondes Bar et 5'Nos est le même.

Pour Ds : :nptII, lorsque l'insertion de l'ADN-T est unique, un fragment est révélé pour les sondes 5'Nos et Intron Actine, et deux fragment sont révélés par la sonde nptII. De plus si le Ds n'est pas excisé, l'un de ces deux fragments est également révélé par la sonde 5'Nos et l'autre par la sonde Intron Actine.

Selon l'invention, les plantes choisies pour les croisements avec la source de transposase sont préférentiellement les plantes issues des événements de transformation monocopies.

### Exemple 2. Croisement avec une lignée R-nj::Ac possédant la transposase Ac insérée dans un gène marqueur phénotypique

### 2.1 Source de transposase R-nj::Ac

La lignée W22/R-nj::Ac contient les gènes constitutifs C2, A1, A2 et les gènes régulateurs C1, pl, B-b. Cette lignée porte donc tous les facteurs requis pour la pigmentation violette dans l'aleurone (identifiée comme normale). Cette lignée possède en plus l'allèle instable R-nj::Ac crée par translocation de chromosome par Dellaporta *et al*. en 1988.

Cette lignée homozygote pour l'allèle R-nj::Ac et utilisée ici est issue de graines provenant du Stock Center (http://www.agron.missouri.edu/) et est nommée As. Les graines présentent des secteurs colorés plus ou moins grands correspondant aux cellules dans lesquelles l'Ac s'est excisé de l'allèle R-nj.

Des analyses moléculaires ont été réalisées sur de l'ADN extrait de feuilles de plantes As homozygotes pour l'allèle R-nj : :Ac, afin d'évaluer l'activité de l'élément Ac inséré dans le gène R-nj. Cette étude de l'état de méthylation des Ac présents dans les lignées As a été réalisée comme pour les lignées A188 et les lignées élites qui servent à la transformation et à l'introgression des gènes d'intérêts. De nombreuses bandes de tailles supérieures à 8 kb sont présentes en haut de chaque piste de tous les échantillons. Elles correspondent à de l'ADN non digéré ou partiellement digéré. Cependant la présence d'une bande de 2.5 kb issue de la digestion par PvuII, une enzyme sensible à la méthylation, indique qu'au moins une copie de l'élément Ac est non méthylée dans le génome de cette lignée As.

### 2.2 Introgression de l'allèle R-nj : :Ac dans d'autres lignées

L'élément R-nj : :Ac peut être avantageusement introgressé dans des lignées connues pour être favorables à la régénération de cals, comme la lignée A188 ( Walbot *et al.,* 1994) ou la lignée Hill. L'élément R-nj : :Ac peut être également introgressé dans des lignées élites, utilisées pour l'introgression des gènes d'intérêts.

Pour vérifier l'activité de l'élément Ac au cours des générations, une analyse phénotypique a été effectuée sur les graines des épis issus de ces croisements. Les épis obtenus par ces croisements présentent des graines variéguées.
Les résultats des croisements entre la lignée As et les lignées élites, ou A188 et M1 montrent que le gène R-nj peut s'exprimer dans d'autres fonds génétiques, soit par la présence des gènes constitutifs nécessaires à l'expression du phénotype dans ces lignées (comme c'est le cas pour A188), soit parce qu'ils sont apportés dans la cellule par la lignée As.

La variégation observée sur les graines suggèrent que l'Ac est toujours actif dans cette génération puisqu'il transpose hors de l'allèle R-nj dans certaines parties des graines issues de ces croisements.

De multiples introgressions réalisées dans les lignées A188 et Hill et dans une lignée élite (jusqu'à 3 croisements dans la lignée, ce qui correspond à environ 93 % de A188 et 7% de W22) ont permis de confirmer ces résultats. Des lignées homozygotes pour l'allèle R-nj : :Ac ont donc été réalisées par autofécondations.

### 2.3 Croisements

La fécondation est réalisée manuellement par une technique connue de l'homme de l'art par le dépôt du pollen de la source de transposase As sur les soies des transformants, de préférence dans le sens plante mâle possédant la transposase dans la plante femelle contenant l'élément Ds : :M.

Une partie de l'épi est parfois prélevée afin d'en extraire des embryons immatures destinés à des protocoles de biologie cellulaire comme la récupération d'embryons ou la callogénèse. La section coupée est préférentiellement recouverte d'un fongicide afin d'éviter les pourritures et les bactéries.

Les embryons prélevés 10 à 15 jours après fécondation sont posés, partie bombée vers le haut sur 25 ml du milieu de régénération RM+ (Ishida *et al,* 1996; Negrotto *et al.,* 2000 ). Les boîtes sont ensuite placées dans une chambre de culture à photopériode régulée (16 h jour - 8 h nuit) et à température constante (23°C). Les plantules sont ensuite acclimatées au phytotron au bout de 10 - 15 jours après mise en culture et avant d'être transférées en serre.

### Exemple 3 : Sélection des événements présentant une excision de l'élément Ds::MS

Les études moléculaires, décrites à l'exemple 1.3, ont permis l'identification d'un événement de transformation monocopie Ds::nptII et d'un événement monocopie Ds::bar utilisés préférentiellement pour évaluer une potentielle excision. Ces études ont également montré que l'élément Ds::M est stable dans la lignée A188 (étude effectuée sur des transformants primaires T0 et premiers descendants T1 par PCR et Southern).

Les études phénotypiques et moléculaires de la lignée As, décrites à l'exemple 2) ont permis de montrer qu'il existait dans leur génome (au moins) une copie de l'élément Ac active capable de s'exciser hors de l'allèle R-nj dans le background génétique initial de la lignée W22 ou dans le background d'une autre lignée.

L'analyse phénotypique de la variégation de l'allèle R-nj sur les épis F1 issus des croisements As X Ds : :M a confirmé que l'élément Ac était toujours actif dans cette génération : la présence de secteurs anthocyanes sur tous les grains matures montre qu'il y a eu production de transposase et excision somatique de l'Ac hors de son site d'insertion initial. L'apport par croisement d'un nouvel élément Ds dans la cellule n'a changé en rien la capacité à suivre l'activité de la source de transposase.

Il est considéré que l'élément Ds n'est pas génétiquement lié à l'allèle R-nj::Ac..

Il reste donc à déterminer si la transposase exprimée par l'élément Ac inséré dans l'allèle R-nj peut permettre l'excision des éléments Ds::bar et Ds::nptII.

### 3.1 A partir de plantes F1 (voie classique)

Des croisements ont été entrepris après l'établissement des lignées monocopies afin de vérifier si les éléments Ds::bar et Ds::nptII sont capables de transposer *in planta.*

Les plantes sources de transposase ont servi de pollinisatrice (en raison d'une semi-stérilité femelle de ces plantes).

### a) recherche par PCR d'excision somatique sur plantes F1 résistantes à l'agent de sélection

Une partie de la descendance F1 issue des croisements entre les plantes Ds : :MS et les plantes As a été semée et de l'ADN a été isolé de jeunes feuilles de chaque plante F1 ainsi générée. L'un des parents étant hémizygote pour l'ADN-T, l'autre étant homozygote pour l'allèle R-nj::Ac, il est attendu que les deux composantes Ac et Ds soient transmises à 50% des plantes F1 issues de ces croisements.

Afin d'identifier les plantes F1 possédant l'ADN-T et de détecter d'éventuelles excisions somatiques, des tests de résistance au marqueur sélectif ont été réalisés sur les jeunes plantes en parallèle avec des tests d'amplification de séquence sur l'ADN de feuilles :
-pour les plantes Ds::npttII

On dépose dans le cornet formé par les feuilles de la plante de 3 à 5 ml d'une solution de kanamycine sulfate 500 mg/l + 0,1% de tween. Les plantes sensibles développent des secteurs chlorotiques sur les parties des feuilles qui ont été en contact avec la solution.
- pour les plantes Ds::bar

On réalise un « leaf painting » en badigeonnant une feuille avec une solution de glufosinate ammonium.

Une première sélection de la résistance à l'antibiotique ou à l'herbicide par une application non destructive a permis de déterminer quelles plantes possédaient le gène marqueur de sélection.

Quand un élément Ds s'excise après production de transposase, il reste un site donneur vide. Une amplification par PCR avec les oligonucléotides EM13 (SEQ ID n°1) et EM14 (SEQ ID n°2) permet de synthétiser un fragment spécifique de 280 pb quand l'ADN-T est délété de l'élément Ds::bar ou Ds::nptII. Une série d'amplifications a donc été réalisée avec le couple EM13-EM14 sur de l'ADN extrait des plantes issues du croisement Ds::nptII X Ac afin de détecter une éventuelle excision somatique dans certaines cellules des échantillons de feuilles prélevées.

L'analyse par PCR de ces plantes F1 a montré qu'il y avait présence d'excision somatique pour toutes les plantes F1 ayant hérité de l'ADN-T et de l'allèle R-nj::Ac. Aucune amplification n'a été détectée sur les plantes ne possédant que l'ADN-T, ni sur les plantes ne possédant aucune des deux composantes.

De plus, toutes les plantes, pour lesquelles une excision somatique a été détectée, se sont révélées être globalement résistantes à l'antibiotique.

Cette bande indique donc la présence d'une excision somatique dans certaines cellules des individus porteurs de l'ADN-T et de l'allèle R-nj::Ac. Conformément à ce qui était prévu, aucun événement d'excision germinale de l'élément Ds::M ne s'est produit pour ces plantes, les deux composantes étant regroupées dans la même cellule depuis trop peu de générations cellulaires (de la fécondation à la jeune plante).

Il aurait fallu que la transposition ait eu lieu dans les gamètes, ou les cellules mères des gamètes, pour pouvoir espérer observer une excision germinale. Ce qui augmente la possibilité d'excision au niveau des gamètes faibles, il aurait donc été étonnant d'obtenir une excision germinale dès la génération F1.

Cependant dans la stratégie envisagée c'est la fréquence d'excision germinale qui est préférentielle pour l'élimination du gène marqueur :
- une excision somatique ne touche généralement pas les gamètes et conduit à la formation de graines et d'individus chimériques dont la plupart des cellules possèdent encore le marqueur d'excision
- une excision germinale touche les cellules, qui vont se différencier dans la voie de la gamétogénèse, et conduit à la formation de graines et d'individus constitués de cellules où le Ds a été excisé (et peut être réinséré) et qui ne possèdent plus le gène marqueur de sélection.

### b) recherche par PCR d'excision germinales potentielles du gène marqueur parmi les plantes sensibles.

C'est pourquoi la recherche d'une excision germinale s'est effectuée sur la génération F2. Les bandes d'amplification spécifique de l'excision ont été dosées pour chaque plante F1, ce dosage permettant de refléter, à travers le taux d'excision somatique, l'activité de la source de transposase.

Les plantes F1 présentant le plus d'excision somatique ont été croisées avec des plantes de la lignée A188 ou avec des plantes d'une lignée élite. Ces croisements ont été réalisés dans les deux sens, les plantes possédant les deux composantes Ac/Ds::M ayant servi de femelles et/ou de mâles pour augmenter les possibilités d'excision germinale par plante F1. Les graines ainsi formées ont été portées à maturation. Des lots de graines F2 provenant de plantes F1 Ds::nptII X Ac résistantes à la kanamycine et de plantes F1 Ds::bar X Ac résistantes au glufosinate et issues de divers croisements ont été semées. Aucun examen phénotypique portant sur la variégation n'est ici réalisé. Les graines exprimant le phénotype ou les graines non pigmentées sont choisies pour le semis avec la même probabilité, pour éviter tout biais.

Une manière simple de tester la fréquence d'excision germinale dans ce système est de sélectionner les plantes F2 pour leur sensibilité à l'agent sélectif et de les tester par analyses moléculaires. Des plantes F2 Ds::bar et des plantes F2 Ds::nptII ont ainsi été testées pour l'amplification par PCR de la bande spécifique d'excision de 280 pb.

Une plante F2 est sensible à l'agent sélectif soit par le biais de la ségrégation, et par conséquent la bande de 280 pb ne doit pas être amplifiée, soit par excision germinale du gène marqueur, et dans ce cas là, la bande spécifique d'excision est détectable par PCR. Cette étude a permis d'amplifier la bande spécifique d'excision à partir de l'ADN de plantes F2 issues des croisements:
- Ds::bar/Ac X A188,
- Ds::bar/Ac X lignée élite,
- lignée élite X Ds::nptII/Ac
- Ds::nptII/Ac X lignée élite.

Des hybridations Southern ainsi que des analyses PCR ont été effectuées sur ces plantes sélectionnées ainsi que sur les plantes initiales T1 Ds::bar et T0 Ds::nptII et les résultats obtenus sont les suivants.

Les analyses par PCR et Southern montrent que les plantes F2 sélectionnées ont hérité d'une partie de l'ADN-T contenant la bordure gauche, les séquences d'intérêt, la cicatrice laissée par l'excision du Ds et la bordure droite, l'élément Ds::M n'a pas été génétiquement transmis aux plantes F2 comme ça l'était pour la F1. Ces plantes F2 sont donc des plantes issues d'événements d'excision germinale de l'élément Ds::M hors de l'ADN-T.

Les fréquences globales d'excision germinale sont variables d'une descendance F2 à l'autre et sont assez faibles. En ce qui concerne le construit Ds::bar, sur les plantes ayant hérité de l'ADN-T, seules quelques plantes ne possèdent plus l'élément Ds::bar, ce qui donne une fréquence d'excision germinale de 0,4%. La fréquence d'excision germinale de l'élément Ds::nptII est un peu plus élevée et atteint 0,7%.

Cette fréquence d'excision germinale est relativement basse. Le phénomène de transition florale est plus rare chez le maïs que chez la tomate ou l'arabette : chez le maïs, il n'y a que deux ou trois méristèmes par individus qui se différencient en organes sexuels et forment les gamètes : un pour la panicule (organe mâle) et un ou deux pour les épis (organes femelles). Il faut donc que l'excision germinale touche, soit un de ces méristèmes avant la gamétogénèse, soit un gamète.

### c) Ségrégation de l'ADN-T et de l'allèle R-nj::Ac .

Une fois que la plante F2 portant l' ADN-T sans le gène marqueur de sélection a été caractérisée, on peut procéder à la ségrégation de la transposase insérée dans R-nj et de l'ADN-T. Cette ségrégation peut avoir lieu au cours de l'introgression de l'ADN-T dans la lignée élite. Il suffit de sélectionner les plantes qui portent l'ADN-T, mais qui n'expriment pas de pigment anthocyane de type navajo. Cette ségrégation peut également être suivie par analyses moléculaires de type Southern grâce aux sondes Sac et S064.

### 3. 2 Méthode de sélection sur cals pour les embryons F1 Ds::nptII X As

Afin d'augmenter la fréquence d'excision germinale, une étape de callogénèse a été réalisée sur des embryons F1 immatures possédant les deux composantes, c'est à dire l'élément Ds::nptII inséré dans l'ADN-T et la source de transposase Ac insérée dans l'allèle R-nj. La lignée choisie comme source de transposase est ici une source homozygote issue de la première introgression de W22/R-nj::Ac dans A188. Le cal possède donc approximativement 75% du génome de A188 et 25% du génome de W22. A188 est une lignée qui permet d'obtenir du cal de type II.

Lors de la croissance du cal et à partir d'une cellule ayant connu un événement d'excision somatique, il est possible de générer un secteur de cal embryogène constitué d'un amas de cellules somaclonales ne possédant plus le gène marqueur de sélection. Une identification phénotypique ou moléculaire de ce secteur permettrait de régénérer un individu sans gène marqueur à partir de ces cellules avec une fréquence plus importante que par la voie classique.

### a) initiation de cals à partir d'embryons immatures F1 sur milieu sélectif et sélection des cals résistants

Des embryons F1 issus des croisements entre les plantes transgéniques Ds::M et les sources de transposase homozygotes pour l'allèle R-nj::Ac ont été prélevés de dix à douze jours après la fécondation, posés stérilement sur un milieu N6 et mis en culture dans une chambre noire afin d'initier la formation de cals indifférenciés de type II selon la méthode d'Armstrong *et al*., 1985.

Après avoir éliminé les axes germinatifs après 15 jours d'initiation, les cals sont maintenus dans l'obscurité à 25°C pendant tout le temps que dure l'induction de cals et la première partie de la multiplication. Les cals commencent à devenir visibles 1 semaine après l'initiation. A chaque sous-culture, c'est à dire toutes les deux ou trois semaines, les parties blanches, embryogènes et friables sont sélectionnées et séparées des parties plus compactes ou muqueuses.

Au bout de 2 à 3 semaines d'initiation, les cals formés sont dupliqués en deux parties :
- une moitié est mise sur un milieu N6 non sélectif, pour continuer la multiplication de cals.
- l'autre moitié est mise sur un milieu N6 contenant de la kanamycine 50 mg/l, pour identifier les cals résistants porteurs de l'ADN-T. La sélection de cals avec la kanamycine nécessite une étape à la lumière, en chambre de culture. L'antibiotique inhibe en effet la synthèse des peptides dans les mitochondries et les chloroplastes. L'inhibition est basée sur la similitude entre la synthèse protéique de ces organites et la synthèse protéique bactérienne. Les cellules végétales sont donc généralement intolérantes aux aminoglycosides comme la kanamycine ou la gentamycine. Mais pour une dose relativement faible, la kanamycine n'est pas destructive et permet une sélection par phénotype colorimétrique: les cals résistants possédant le gène nptll verdissent quand ils sont exposés à la lumière (les chloroplastes fonctionnels accumulent la cholorophylle), alors que les cals sensibles restent jaunes.

Cette étape de sélection permet d'identifier les cals qui possèdent l'ADN-T et l'élément Ds::nptII et d'éliminer les cals qui ne possèdent dans leur génome que la composante R-nj::Ac (dans le cas d'une source de transposase homozygote).

### b) multiplication des cals résistants sur milieu non sélectif

Les parties des cals résistants qui ont été dupliquées et mises sur milieu non sélectif sont cultivées à l'obscurité. Il y a donc multiplication des cellules qui possèdent les deux composantes. Au cours de cette multiplication, il va y avoir production de transposase et un certain nombre de cellules vont être issues d'événements d'excision de l'élément Ds::nptII hors de l'ADN-T. Ces cellules en se multipliant vont conduire à la formation de cals chimériques constitués de secteurs d'excision somatique du Ds::nptII et de secteurs sans excision.

Les cals sont ensuite mis à la lumière, pendant une semaine. Les parties jaunes sont grossièrement isolées.

### c) régénération de plantules à partir d'un secteur d'excision

Les bouts de cals isolés sont mis à régénérer sur un milieu non sélectif en chambre de culture et à la lumière. Des plantes sont ainsi régénérées à partir des différentes parties embryogènes et potentiellement à partir d'un secteur d'excision de l'élément Ds::nptll.

### d) recherche d'excision germinale sur les plantes sensibles

Les plantes, lorsqu'elles ont atteint le stade trois feuilles sont traitées avec une solution de kanamycine sulfate. Les plantes présentant des secteurs chlorotiques sont donc des plantes sensibles à l'agent sélectif qui sont issues d'un cal initialement résistant. Parmi les plantes testées, certaines plantes originaires du même embryon se sont avérées être sensibles à la kanamycine.

Des analyses PCR et Southern ont en effet montré que l'élément Ds::nptll avait été excisé de son emplacement initial et ne s'était pas réinséré. La fréquence d'excision est donc considérablement augmentée par le biais de la culture de cals et atteint 8% des plantes ainsi régénérées.

Un séquençage des cicatrices laissées sur l'ADN-T par l'excision de l'élément Ds::nptII a été effectué pour les plantes générées par la voie reproductrice et par la voie végétative. L'analyse des séquences obtenues montre que certaines plantes de la voie reproductrice sont issues de deux événements d'excision germinale différents bien qu'issues du même croisement. Par contre les séquences des cicatrices pour les plantes obtenues par callogénèse sont identiques. Ce qui confirmerait que ces plantes sont issues du même évènement d'excision.

### 3.3 Méthode améliorée de sélection d'événements d'excision germinale en génération F1

Selon un mode de réalisation préféré de l'invention, la cassette d'expression comprenant une séquence nucléotidique codant pour un marqueur de sélection (ii) comprend également, à l'intérieur de l'élément Ds, un autre gène marqueur phénotypique de type gène rapporteur non destructif. Ce gène rapporteur peut être par exemple le gène codant pour une protéine fluorescente verte GFP (Nielsen et al. 1999), dont la détection de l'expression dans les tissus n'est pas destructive : l'observation est réalisée sous une loupe équipée d'un éclairage à longueur d'onde spécifique. Lorsque l'élément Ds s'excise, le gène GFP n'est plus présent, et les secteurs d'excision somatique sont repérables sous la « loupe GFP » car ils ne fluorescent plus.

Dans le cadre de cet exemple, le plasmide pBios491 est dérivé du plasmide pBios340 (Ds ::nptII) décrit à l'exemple 1a, par une insertion, à l'intérieur de l'élément Ds et dans le même cadre de lecture que la cassette NptII, d'une cassette d'expression contenant le promoteur CsVMV du virus de la mosaïque des nervures de manioc (WO 97/48819), le premier intron du gène actine 1 de riz, le cadre de lecture ouvert GFP (Nielsen et al. 1999) et le terminateur Nos.

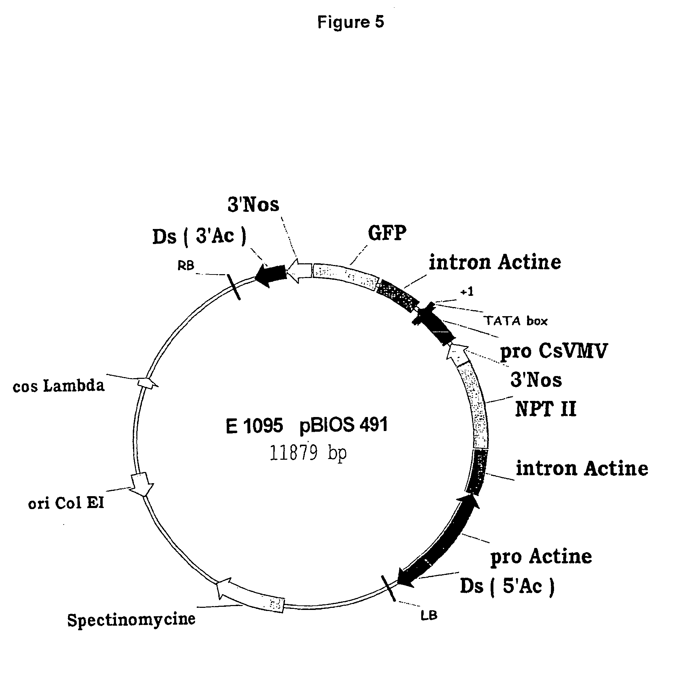

Le Ds ::GFP-nptII a une taille de 5473 pb (Figure n° 5).

Comme décrit dans les exemples 3.1 et 3.2, la sélection des événements d'excision germinale peut suivre les étapes suivantes :
- les embryons T1 issus du croisement entre les transformants portant la cassette gène d'intérêt -Ds : :MS et la source de transposase sont récupérés 10 jours après la pollinisation.
- ces embryons sont prélevés et sont mis en culture sur un milieu de callogénèse non sélectif à l'obscurité pendant 15 à 30 jours.
- les cals sont alors observés sous loupe GFP et la fraction non fluorescente du cal, correspondant aux secteurs d'excision somatique, est alors prélevée pour multiplication et régénération de la plante. Les plantes ainsi produites contiennent le gène d'intérêt sans séquence auxiliaire étrangère.

L'intérêt de ce vecteur Ds :: GFP-MS dans le cadre de l'invention est donc multiple ; en effet :
- l'identification des embryons immatures F1 porteurs du transgène, qui fluorescent, se fait par simple observation visuelle sous la loupe GFP ;
- la callogénèse à partir des embryons sélectionnés peut se faire sur un milieu non sélectif afin d'induire de nombreux départs de cals et ainsi favoriser le départ de zones d'excision somatique dépourvues du gène de sélection.
- la sélection des cals issus de cellules où il y a eu excision somatique est facilitée sous la loupe à fluorescence ; de plus ces cals peuvent être directement et précisément prélevés afin d'être mis en régénération
- une fois les plantes régénérées, l'absence de fluorescence (excision somatique) peut aussi être confirmée.

### 4. Production de plantes mâles stériles dépourvues de gène marqueur kanamycine

### 4.1 Préparation des construits A9-barnase- Ds ::nptII

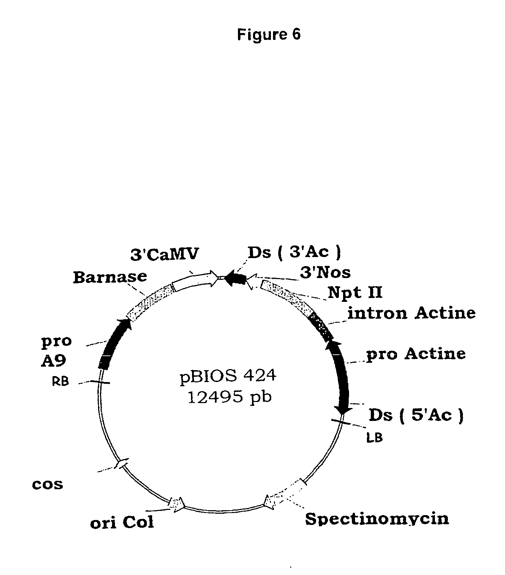

Le plasmide pBios424 est dérivé du plasmide pBios340 décrit à l'exemple 1a par insertion de la cassette 'gène d'intérêt' comprenant le promoteur A9 (WO 92 11379), correspondant à la région 5' non codante du gène A9 d'*Arabidopsis thaliana,* le cadre de lecture ouvert du gène barnase (Hartley, 1988 ; Gene Bank n°X 12871) et le terminateur 3'CaMV (Franck et al. 1980 ; Gene Bank n° V 00141), en dehors de l'élément Ds. Le gène barnase, qui confère la stérilité mâle, code une Ribonucléase (RNase). Ce gène a été isolé à partir de *Bacillus amyloliquefasciens* et est décrit dans la publication de Hartley (1988).
Le A9-barnase-Ds ::nptII a une taille de 12495 pb (Figure 6).

Le plasmide superbinaire pRec424 utilisé pour la transformation est obtenu par recombinaison homologue entre un vecteur intermédiaire dérivé du plasmide pBios424 et le vecteur pSB1 de Japan Tobacco (EP 672 752), comme décrit à l'exemple 1b.

### 4.2 Sélection des transformants résistants et analyse moléculaire

7 événements de transformation ont été produits dont 5 ont été sélectionnés comme étant mâles stériles (A9-barnase) et résistants à la kanamycine (NptII).

L'analyse moléculaire de ces évènements a été réalisée pour déterminer les insertions simples. Cette analyse permet de caractériser les événements de transformation retenus en terme de nombre de copies de l'ADN-T intégré et du nombre de loci d'intégration mis en jeu. Pour cela différentes sondes moléculaires sont utilisées.

L'ADN génomique des plantes a été digéré par l'enzyme de restriction *Nco*I, séparé par électrophorèse sur gel d'agarose et transféré sur membrane de nylon (membranes Hybond N+) en vue de l'hybridation avec les différentes sondes moléculaires.

### Choix des sondes moléculaires :

- L'utilisation des sondes Promoteur A9 (S007, SEQ ID NO:7), Bamase (S032, SEQ ID NO:8), Intron actine, couplée à la restriction enzymatique *Nco*I, permet de caractériser le profil moléculaire des événements de transformation.
- La séquence du plasmide de base étant connue, des couples d'oligonucléotides spécifiques des régions plasmidiques situées à l'extérieur de l'ADN-T ont été synthétisés puis utilisés comme amorces pour générer par PCR une sonde extrabordure droite (RB) et une sonde extrabordure gauche (LB). Du côté droit, à 40 paires de bases de la bordure droite, l'amplification PCR a généré une sonde de 353 paires de bases et du côté gauche, à 29 paires de bases de la bordure gauche, l'amplification a généré une sonde de 299 paires de bases. La sonde appelée "sonde extrabordures" est constituée du mélange des deux sondes décrites ci-dessus. Son utilisation permet de repérer les plantes ne présentant pas d'intégration de régions du plasmide proches de l'ADN-T.

La taille des signaux attendus après hybridation avec les différentes sondes pour une insertion unique de l'ADN-T non tronquée et lorsqu'il n'y a pas de dépassement de bordure est la suivante :

| Enzyme de restriction | Nombre de copies | Taille des signaux attendus | | | |
|---|---|---|---|---|---|
| | | Sonde promoteur A9 (pro A9) | Sonde Intron actine | Sonde barnase | Sonde extrabordures |
| *Nco*I | 1 | ≥ 1189 pb | ≥ 2550 pb | 2733 pb | Pas de signal |

Le bilan des signaux observés sur les transformants primaires montre que les 5 événements présentent des profils d'insertion simples (1 ou 2 copies).

### 4.3 Croisement avec une lignées R-nj ::Ac et sélection des événements présentant une excision de l'élément Ds ::MS

Les transformants primaires présentant des profils d'insertion simple ont été croisés avec la source de transposase homozygote pour l'allèle Rnj-Ac comme décrit à l'exemple 2.

Les embryons correspondants à la génération T1 (ou F1) ont été prélevés 15 jours après la fécondation, et mis en culture in vitro sur du milieu de culture contenant 50 mg de Kanamycine.

Une analyse PCR avec des oligonucléotides adéquats peut permettre de vérifier si ces événements d'excision somatique ont eu lieu dans les T1 sélectionnées.
Les échantillons d'ADN des plantes T0 et T1 ont été soumis à une amplification PCR à l'aide des oligonucléotides Barn5 (SEQ ID NO:6) et EM11 (SEQ ID NO:5). Si le Ds ::*nptII* est excisé du T-DNA, un fragment de 760 pb peut être amplifié. S'il n'est pas excisé le fragment de 4160 pb qui devrait être amplifié, n'apparaît pas dans ces conditions de PCR. La bande d'amplification de 760 pb révélant l'excision du Ds::*nptII* est présente dans la plupart des échantillons de plantes T1 mais pas dans les T0. Aucune excision somatique n'a été mise en évidence dans les T0, ce qui démontre que l'excision est bien maîtrisée. Le gel d'électrophorèse a été transféré sur membrane en nylon, pour être hybridé avec une sonde 3'CaMV afin de confirmer l'identité du fragment amplifié.
Les expériences de PCR ont donc bien permis de confirmer la présence d'excision somatique dans la plupart des plantes T1 portant les 2 constituants : Ds :*:nptII* et transposase.

Après sortie d'*in vitro* les plantes T1 sont acclimatées au phytotron puis en serre. A la floraison, les plantes T1 sélectionnées sont croisées avec une lignée élite, définie comme étant une lignée présentant un potentiel agronomique et commercial important, à une période donnée. Selon la méthode de l'invention, des événements d'excision germinale peuvent être trouvés en criblant un grand nombre de F2 issues de ces lignées T1.

Amorces utilisées pour détecter l'excision des éléments Ds::Bar et Ds::npt II

| **Nom** | **SEQ ID n°** | **Séquences** |
|---|---|---|
| EM 13 | 1 | 5'-CGACAATCTGATCATGAGCG-3' |
| EM14 | 2 | 5-CTTAATAACACATTGCGGACG-3' |
| Barn5 | 6 | 5'-GGTTTCGCTCATGTGTTGAGC-3' |
| EM11 | 5 | 5'-CATTGCGGACGTTTTTAATGTACTG-3' |

Amorces utilisées pour détecter les transformants Ds::npt II

| **Nom** | **SEQ ID n°** | **Séquences** |
|---|---|---|
| Actint1 | 3 | 5'-CGAATTCGCGCCGGTAAC -3' |
| Actint2 | 4 | 5'-TCGTGCCGARTTCGATATCAAGC -3' |

Les sondes utilisées pour les Southern blots sont données ci-dessous

| **Nom** | **Méthodes d'obtention** | **Séquence cible** | **Taille** |
|---|---|---|---|
| S006 | Fragment de restriction : *Sma*I-*Sma*I | Gène bar | 600pb |
| S063 | Fragment PCR : couple d'oligonucléotides Actint1-Actint2 | Intron Actine | 480 pb |
| S064 | Fragment PCR : couple d'oligonucléotides 5'Nos1-5'Nos2 | 5'pNos | 130 pb |
| S020 | Fragment PCR : couple d'oligonucléotides kana7-kana8 | Gène *npt II* | 1000 pb |
| SAc | Fragment de restriction : HindIII-HindIII | Gène *Ac* | 1605 pb |
| S007 | Fragment de restriction: HindIII-HindIII | Barnase | 900 pb |
| S032 | Fragment de restriction: XbaI/ XhoI | Promoteur A9 | 1000 pb |

### BIBLIOGRAPHIE

**An et al.** (1986), Plant Physiol., 81 : 86-91
**Anderson O.D. et al.** (1989),T.A.G., 77:689-700
**Armstrong et al.** (1996) , Planta, 164 :207-214
**Bechtold et al.** (1993), Comptes rendus Académie des Sciences Paris, Serie 3,316:1194-1199
**Bevan et al.** (1983), VAR II :369-379
**Brink et al.** (1973), Genetics, 73 : 273-296
**Chalfie et al.** (1994), Science, 263 :802-807
**Chesson A. et al.** (2000), La Recherche, 327 :27-44
**Chopra S. et al.** (1999), PNAS, USA 96 :15330-15335
**Chernyshev et al.** (1988), Genetika, 24 :1338-1344
**Chupeau et al.** (1989), Biotechnology, 7(5):503-508
**Dellaporta S. *et* al.** (1988), Plenum Press, pp 263-282
**Depicker et al.** ( 1982), Mol.Appl. Genet. 1 : 561-573
**Depigny-This et al.** (1992), Plant Mol. Biol., 20:467-479
**Fedoroff N.V. et al.** (1983), Cell, 35 :243-251
**Finer et al.** (1992). Plant Cell Report, 11:323-328
**Flavell R.B. et al.** (1992), Bio/Technology, 10 : 141-144
**Franck et al.** (1980) *Cell,* 21, 285-294
**Fromm M. et al.** (1990), Biotechnology, 8:833-839
**Grotewold E. et al.** (1998), Plant Cell, 10 :74-740
**Guerche et al.** (1987), Mol. Gen. Genet., 206:382
**Hartley, R.W** (1988) J. Mol. Biol. 202, 913-915
**Herrera-Estrella et al** (1983), EMBO J. 2, 987-995
**Hood E.E. et al.** (1986), Journal of Bacteriology, 168 :1291-1301
**Hood E.E. et al.** (1993), Trans. Res., 2 :208-218
**Ishida et al**.(1996), Nature Biotechnology, 14 : 754-750
**Jefferson et al.** (1987), Plant Mol. Biol., 5 :387-405
**Kay et al.** (1987), Science, 236:4805
**Lazo G.R. et al.** (1991), Biotechnology (NY), 9(10) :963-967
**Lechelt et al.** (1989), Mol. Gen. Genest, 219 :225-234
**Ludwig S. et al.** (1989), PNAS of USA, 86 :7092-7096
**Lyzrik L. et al.** (1997), Nucleic Acids Research, 44 :123-132
**MacRae A. et al.** (1994), Genetica, 92 (2) : 77-89
**Mannimem I. et al.** (1993), Plant Mol Biol, 22 :829-846
**Mc Elroy et al.** (1991), Mol. Gen. Genet., 231 :150-160
**Mototashi R. et al.** (1996), Mol Gen Genet, 25 :148-152
**Müller-Neumann et al.** (1984), Mol Gen Genet, 228 :201-208
**Negrotto D. et al.** (2000), Plant cell Rep., 19 :798-803
**Neuhaus et al.** (1987), Theoretical and applied Genet., 75(1):30-36
**Nielsen K. et al.** (1999) Physiological and Molecular Plant Pathology, 54, 1-12 **Ooms G. et al.** (1982), Plant Molecular Biology, 1 :265-271
**Pereira et al.** (1986), EMBO, J5 :835-841
**Roberts et al.** (1989), Plant Cell, 1 : 569-578
**Schocher et al.** (1986). Biotechnology, 4:1093-1096
**Seth J. Davis** (1998), Plant Molecular Biology, 36: 521-528.
**Yoder J.T. et al.** (1993), Bio/Technology, 12:263-266
**Walbot V. et Bodeau et al.** (1994), Maize News Letter, 68: 98-99
**Watson et al.** (1994), Ed. De Boeck Université, pp 273-292
**Weising K. et al.** (1995), CRC Press, Boca Raton. FL.
**White et al.** (1990), NAR 18 :1062

## Revendications

1. Méthode d'obtention d'une plante monocotylédone transgénique contenant un gène d'intérêt (i) sans séquence auxiliaire étrangère, comprenant les étapes suivantes:
a) transformation d'au moins une cellule de plante ne possédant aucune transposase active, avec un vecteur comprenant deux cassettes d'expression, l'une comprenant une séquence nucléotidique d'intérêt (i), l'autre comprenant une séquence nucléotidique codant pour un marqueur de sélection (ii) encadrée par les séquences mobilisables d'un transposon, ladite cassette d'expression comprenant une séquence nucléotidique d'intérêt (i) étant en dehors de l'élément transposon;
b) sélection des plantes transformées avec le marqueur de sélection (ii);
c) croisement d'une plante transformée avec une autre plante appartenant à une lignée contenant dans son génome un gène, codant pour une transposase active endogène, et qui se trouve au milieu d'un marqueur phénotypique d'excision (iii), pour obtenir une F1 ou tout autre individu de génération ultérieure ;
d) sélection des cellules ou des individus porteurs du gène d'intérêt sans séquence auxiliaire étrangère, à partir de la génération F1 ;
e) régénération de plantes à partir des cellules ou des individus sélectionnés en (d).

2. Méthode selon la revendication 1, dans laquelle les séquences auxiliaires étrangères codent pour un gène marqueur de sélection (ii) conférant une résistance à un antibiotique ou à un herbicide, comme le gène nptII ou le gène bar.

3. Méthode selon la revendication 1, dans laquelle les séquences auxiliaires étrangères codent pour un gène marqueur phénotypique (ii).

4. Méthode selon l'une des revendications 1 à 3, dans laquelle les séquences mobilisables d'un transposon encadrent, en plus de la cassette d'expression comprenant une séquence nucléotidique codant pour un marqueur de sélection (ii), une seconde cassette d'expression comprenant une séquence nucléotidique codant pour un gène rapporteur à sélection non destructive

5. Méthode selon la revendication 4, dans laquelle la séquence nucléotidique codant pour un gène rapporteur à sélection non destructive code pour une GFP.

6. Méthode selon l'une des revendications 1 à 5, dans laquelle la plante monocotylédone transgénique est une plante de maïs.

7. Méthode selon l'une des revendications 1 à 6, dans laquelle le système transposon est le système Ac/Ds.

8. Méthode selon l'une des revendications 1 à 7, dans laquelle les individus à l'étape (d) peuvent être des plantes F1, des plantes F2 ou des cals.

9. Méthode selon la revendication 6, dans laquelle la plante transformée appartient à la lignée A188.

10. Méthode selon l'une des revendications 6 à 9, dans laquelle la plante transformée est croisée à l'étape (c) avec une autre plante, dans laquelle l'élément Ac actif est situé dans une région chromosomique telle que l'excision dusit élément Ac se traduit par la production de secteurs anthocyanés sur la couronne de la graine, dont l'embryon.

11. Méthode selon l'une des revendications 1 à 10, dans laquelle la sélection des individus à l'étape (d) se fait par voie reproductive, comprenant les étapes suivantes:
- sélection des grains F1 variégués;
- sélection des événements d'excision somatique de l'élément transposon défectif de type Ds contenant le gène de sélection (nommé Ds::M) par technique PCR;
- sélection des événements d'excision germinale de l'élément Ds::M par technique PCR ;
- obtention de semis de plantes F2 à partir de ces événements.

12. Méthode selon l'une des revendications 1 à 10, dans laquelle la sélection des individus à l'étape (d) se fait par voie végétative, comprenant les étapes suivantes:
- production de cals à partir d'embryons immatures F1
- sélection visuelle des cals contenant l'ADN-T et l'élément transposon défectif de type Ds contenant le gène de sélection (nommé Ds::M)
- multiplication de cals et recherche de secteurs d'excision de l'élément Ds::M
- régénération de plantes F1 à partir de ces secteurs d'excision.

13. Méthode selon la revendication 10 pour l'obtention de plantes F1 directement régénérées à partir de cals sélectionnés, par la voix de culture *in vitro* d'embryons immatures d'épis F1.

## Claims

1. Method for obtaining a transgenic monocotyledon plant containing a gene of interest (i) free of foreign ancillary sequence, comprising the following steps:
a) transforming at least one cell of a plant having no active transposase with a vector comprising two expression cassettes, one comprising a nucleotide sequence of interest (i), the other comprising a nucleotide sequence encoding a selection marker (ii) flanked by the mobilizable sequences of a transposon, said expression cassette comprising a nucleotide sequence of interest (i) being outside the transposon element;
b) selecting the transformed plants with the selection marker (ii);
c) crossing a transformed plant with another plant belonging to a line containing in its genome a gene encoding an endogenous active transposase, and which is in the middle of a phenotypic marker for excision (iii), so as to obtain an F1 or any other individual of a subsequent generation;
d) selecting the cells or the individuals carrying the gene of interest free of foreign ancillary sequence, from the F1 generation;
e) regenerating plants from the cells or the individuals selected in (d).

2. Method according to Claim 1, in which the foreign ancillary sequences encode a selection marker gene (ii) conferring resistance to an antibiotic or a herbicide, such as the nptII gene or the bar gene.

3. Method according to Claim 1, in which the foreign ancillary sequences encode a phenotypic marker gene (ii).

4. Method according to one of Claims 1 to 3, in which the mobilizable sequences of a transposon flank, in addition to the expression cassette comprising a nucleotide sequence encoding a selection marker (ii), a second expression cassette comprising a nucleotide sequence encoding a reporter gene with nondestructive selection.

5. Method according to Claim 4, in which the nucleotide sequence encoding a reporter gene with nondestructive selection encodes a GFP.

6. Method according to one of Claims 1 to 5, in which the transgenic monocotyledon plant is a maize plant.

7. Method according to one of Claims 1 to 6, in which the transposon system is the Ac/Ds system.

8. Method according to one of Claims 1 to 7, in which the individuals in step (d) may be F1 plants, F2 plants or calluses.

9. Method according to Claim 6, in which the transformed plant belongs to the A188 line.

10. Method according to one of Claims 6 to 9, in which the transformed plant is crossed, in step (c), with another plant, in which the active element Ac is sited in a chromosomal region such that excision of the said element Ac results in the production of anthocyan-containing sectors on the crown of the seed, including the embryo.

11. Method according to one of Claims 1 to 10, in which the selection of the individuals, in step (d), is carried out via the reproductive pathway, comprising the following steps:
- selecting the variegated F1 seeds;
- selecting the events of somatic excision of the Ds-type defective transposon element containing the selection gene (called Ds::M), by the PCR technique;
- selecting the events of germinal excision of the Ds::M element, by the PCR technique;
- obtaining F2 plant sowing based on these events.

12. Method according to one of Claims 1 to 10, in which the selection of the individuals, in step (d), is carried out via the vegetative pathway, comprising the following steps:
- producing calluses from immature F1 embryos,
- visually selecting the calluses containing the T-DNA and the Ds-type defective transposon element containing the selection gene (called Ds::M),
- multiplying calluses and searching for sectors of excision of the Ds::M element,
- regenerating F1 plants from these sectors of excision.

13. Method according to Claim 10, for obtaining F1 plants directly regenerated from selected calluses, by *in vitro* culturing of immature embryos of F1 ears.

## Patentansprüche

1. Verfahren zur Herstellung einer monokotylen Pflanze, welche ein gewünschtes Gen (i) ohne zusätzliche Fremdsequenz enthält, wobei das Verfahren die folgenden Schritte umfaßt:
a) Transformation von mindestens einer Pflanzenzelle ohne aktiver Transposase mit einem Vektor, der zwei Expressionskassetten enthält, wobei die eine eine gewünschte Nukleotidsequenz (i) enthält und die andere eine Nukleotidsequenz enthält, die für einen Selektionsmarker (ii) codiert und die von den mobilisierbaren Sequenzen eines Transposons eingerahmt ist, wobei die Expressionskassette, die eine gewünschte Nukleotidsequenz (i) enthält, außerhalb des Transposonelements liegt;
b) Selektion von transformierten Pflanzen mit dem Selektionsmarker (ii);
c) Kreuzen einer transformierten Pflanze mit einer anderen Pflanze, die zu einer Linie gehört, die in ihrem Genom ein Gen enthält, das für eine endogene aktive Transposase codiert und das innerhalb eines phänotypischen Exzisionsmarkers (iii) liegt, wodurch man zu einer F1 oder eines sonstigen Einzelorganismus einer Folgegeneration gelangt;
d) Selektion von Zellen oder Einzelorganismen, die das gewünschte Gen ohne zusätzliche Fremdsequenz enthalten, wobei von der F1-Generation ausgegangen wird;
e) Regeneration von Pflanzen aus dem in (d) selektierten Zellen oder Einzelorganismen.

2. Verfahren nach Anspruch 1, wobei die zusätzlichen Fremdsequenzen für ein Selektionsmarkergen (ii) codieren, das eine Resistenz gegen ein Antibiotikum oder ein Herbizid vermittelt, wie das nptII-Gen oder das bar-Gen.

3. Verfahren nach Anspruch 1, wobei die zusätzlichen Fremdsequenzen für ein phänotypisches Markergen (ii) codieren.

4. Verfahren nach einem der Ansprüche 1 bis 3, wobei die mobilisierbaren Transposonsequenzen zusätzlich zu der Expressionskassette, die eine Nukleotidsequenz, die für einen Selektionsmarker (ii) codiert, enthält, eine zweite Expressionskassette, die eine Nukleotidsequenz, die für ein Reportergen für die nichtdestruktive Selektion codiert, enthält, einrahmen.

5. Verfahren nach Anspruch 4, wobei die Nukleotidsequenz, die für ein Reportergen für die nichtdestruktive Selektion codiert, für ein GFP codiert.

6. Verfahren nach einem der Ansprüche 1 bis 5, wobei es sich bei der transgenen monokotylen Pflanze um eine Maispflanze handelt.

7. Verfahren nach einem der Ansprüche 1 bis 6, wobei es sich bei dem Transposonsystem um das Ac/Ds-System handelt.

8. Verfahren nach einem der Ansprüche 1 bis 7, wobei es sich bei den Einzelorganismen im Schritt (d) um F1-Pflanzen, F2-Pflanzen oder Kalli handeln kann.

9. Verfahren nach Anspruch 6, wobei die transformierte Pflanze zur Linie A188 gehört.

10. Verfahren nach einem der Ansprüche 6 bis 9, bei dem die transformierte Pflanze im Schritt (c) mit einer anderen Pflanze gekreuzt wird, in der sich das aktive Ac-Element in einer Chromosonenregion befindet, sodaß sich die Exzision dieses Ac-Elements durch Produktion von anthozyanhaltigen Abschnitten im Kronenteil des Korns, darunter auch den Embryo, ausdrückt.

11. Verfahren nach einem der Ansprüche 1 bis 10, wobei diese Selektion von Einzelorganismen in Schritt (d) auf reproduktivem Weg erfolgt, wobei das Verfahren die folgenden Schritte umfaßt:
- Selektion von panaschierten F1-Körnern;
- Selektion von somatischen Exzisionsereignissen des defektiven Ds-Typ-Transposonelements, das das Selektionsgen enthält (mit der Bezeichnung Ds::M), mittels PCR-Technik;
- Selektion von Exzisionsereignissen des Ds::M-Elements aus der Keimbahn;
- Gewinnung von Samen von F2-Pflanzen ausgehend von diesen Ereignissen.

12. Verfahren nach einem der Ansprüche 1 bis 10, bei der die Selektion von Einzelorganismen in Schritt (d) auf vegetativem Weg erfolgt, wobei das Verfahren die folgenden Schritte umfaßt:
- Produktion von Kalli aus unreifen F1-Embryonen,
- optische Selektion von Kalli, die die T-DNA und das defektive Ds-Typ-Transposonelement, das das Selektionsgen enthält (mit der Bezeichnung Ds::M), enthalten,
- Vermehrung der Kalli und Suche nach Abschnitten, in denen eine Exzision des Elements Ds::M stattgefunden hat,
- Regeneration von Fl-Pflanzen aus diesen Exzisionssektoren.

13. Verfahren nach Anspruch 10 zur Gewinnung von F1-Pflanzen, die direkt ausgehend von selektierten Kalli regeneriert werden, und zwar mittels invitro-Kultur von unreifen Embryonen von Fl-Ähren.


### LISTE DE SEQUENCES

<110> RhoBio SA
<120> Méthode d'obtention de plante Monocotylédone contenant un gène d'intérêt sans séquence auxilière étrangère
<130> PM 01015
<140>
   <141>
<150> FR 0107597
   <151> 2001-06-11
<160> 8
<170> PatentIn Ver. 2.1
<210> 1
   <211> 20
   <212> ADN
   <213> Séquence artificielle
<220>
   <223> Description de la séquence artificielle: Oligonucléotide synthétique
<400> 1
   cgacaatctg atcatgagcg 20
<210> 2
   <211> 21
   <212> ADN
   <213> Séquence artificielle
<220>
   <223> Description de la séquence artificielle: Oligonucléotide synthétique
<400> 2
   cttaataaca cattgcggac g 21
<210> 3
   <211> 18
   <212> ADN
   <213> Séquence artificielle
<220>
   <223> Description de la séquence artificielle: Oligonucléotide synthétique
<400> 3
   cgaattcgcg ccggtaac 18
<210> 4
   <211> 23
   <212> ADN
   <213> Séquence artificielle
<220>
   <223> Description de la séquence artificielle: Oligonucléotide synthétique
<400> 4
   tcgtgccgaa ttcgatatca agc 23
<210> 5
   <211> 25
   <212> ADN
   <213> Séquence artificielle
<220>
   <223> Description de la séquence artificielle: Oligonucléotide synthétique EM11
<400> 5
   cattgcggac gtttttaatg tactg 25
<210> 6
   <211> 21
   <212> ADN
   <213> Séquence artificielle
<220>
   <223> Description de la séquence artificielle: Oligonucléotide synthétique Barn5
<400> 6
   ggtttcgctc atgtgttgag c 21
<210> 7
   <211> 985
   <212> ADN
   <213> Arabidopsis thaliana
<400> 7
   ctcgaggtcg acggtatcga taagcttgca tgcctgcagg tagacattgt aggttggttt 60
   tgatgatgat aagtaatcat tggagaattg tctaacacat gcactggaga attattgact 120
   ctaccacgtt ctctttgata ttcctcgatt ttcctcgtga tttcatcagc ctctccgaaa 180
   aagtaattgt atccactaga actttgggaa tctcccatct aatttatgta ttagagaagt 240
   tataatattt tggggaaata gattttctct actgattttg ttgtgtgaca ttatattttt 300
   ataagtacat gtttctgttt cgttatattg ttgtcgtggt tgagtcttta ttagagcatg 360
   taaatatgtt tatgaaataa gcgagaaagg aattaattaa acgtatcgag tgataaatgc 420
   tttaatggat tcgagattta gtattcttaa atttttgttt cattatcatt gattataaaa 480
   ctaagttatg ttgatctcaa atccttaatt atgttctcct aagaagagta caagtggtgg 540
   gaacgaaaga tgagtaaaat actaaaaatc ttttctcaaa agtcaaatcg cattagttaa 600
   caaaaacaaa ccatgtgtta ccgtcaaatc aatgtgttta aaagatgtta accactaatc 660
   aagcatttac gtgtaaccgg atcaaccgga tttgggtttt gaatatgttg tggagatgta 720
   tataaatgat aaattaattg aatatcttaa ttaatctgtg aaagaaacta catcacacac 780
   tttgttattt cccctagctt ttagtttttt tatcatgcaa aacttatgaa gtaactagat 840
   caagatcaca aaaaaaaagc atcacttcac ttcatgacct aattattctc gaagcccaaa 900
   actatttaca tacactttta ttctataaat atagatgatg gaattcacca atccaaaagt 960
   gaataaaaaa cacaagtact ctaga 985
<210> 8
   <211> 898
   <212> ADN
   <213> Bacillus amyloliquefaciens
<400> 8
   aagcttctag accatggcac aggttatcaa cacgtttgac ggggttgcgg attatcttca 60
   gacatatcat aagctacctg ataattacat tacaaaatca gaagcacaag ccctcggctg 120
   ggtggcatca aaagggaacc ttgcagacgt cgctccgggg aaaagcatcg gcggagacat 180
   cttctcaaac agggaaggca aactcccggg caaaagcgga cgaacatggc gtgaagcgga 240
   tattaactat acatcaggct tcagaaattc agaccggatt ctttactcaa gcgactggct 300
   gatttacaaa acaacggacc attatcagac ctttacaaaa atcagataac gaaaaaaacg 360
   gcttcctgcg gaggccgttt ttttcagctt tacataaagt gtgtaataaa tttttcttca 420
   aactctgatc ggtcaatttc actttccgga tccggtccaa tctgcagccg tccgagacag 480
   gaggacatcg tccagctgaa accggggcag aatccggcca tttctgaaga gaaaaatggt 540
   aaactgatag aataaaatca taagaaagga gccgcacatg aaaaaagcag tcattaacgg 600
   ggaacaaatc agaagtatca gcgacctcca ccagacattg aaaaaggagc ttgcccttcc 660
   ggaatactac ggtgaaaacc tggacgcttt atgggattgt ctgaccggat gggtggagta 720
   cccgctcgtt ttggaatgga ggcagtttga acaaagcaag cagctgactg aaaatggcgc 780
   cgagagtgtg cttcaggttt tccgtgaagc gaaagcggaa ggctgcgaca tcaccatcat 840
   actttcttaa tacgatcaat gggagatgaa caatatggaa acacaaaccc gcaagctt 898